# EUROPÄISCHE PATENTSCHRIFT

(11) **EP 4 282 549 B1**
(45) Veröffentlichungstag und Bekanntmachung des Hinweises auf die Patenterteilung: **26.03.2025**
(21) Anmeldenummer: 22174840.3
(22) Anmeldetag: 23.05.2022
(51) Int. Cl.: B09B 3/70, B09B 5/00, C12M 1/00, B01J 8/00, B01J 19/24, A61G 17/00, A61G 13/00, B09B 101/65

(54) **VORRICHTUNG UND VERFAHREN ZUR ALKALISCHEN HYDROLYSE VON LEICHNAMEN UND/ODER KADAVERN**
DEVICE AND METHOD FOR ALKALINE HYDROLYSIS OF CORPSES AND / OR CADAVERS
DISPOSITIF ET PROCÉDÉ D'HYDROLYSE ALCALINE DES CADAVRES ET/OU DE CARCASSES

(43) Veröffentlichungstag der Anmeldung: 29.11.2023
(73) Patentinhaber: Krematorium Am Waldfriedhof Schwäbisch Hall GmbH & Co. KG, 74523 Schwäbisch Hall (DE)
(72) Erfinder: Lutz, Jochen, 74538 Rosengarten (DE); Lutz, Patrick, 74538 Rosengarten (DE); Böhm, Simon, 74523 Schwäbisch Hall (DE)
(74) Vertreter: Jakelski & Althoff Patentanwälte PartG mbB

(56) Entgegenhaltungen:
- WO-A1-2022/011427
- DE-U1- 202021 102 871
- US-A1- 2011 130 612

## Beschreibung

Die vorliegende Erfindung betrifft die alkalische Hydrolyse - auch als Verseifung oder Aquamation bezeichnet - von Leichnamen und Kadavern. Die alkalische Hydrolyse kann zur Bestattung des Leichnams bzw. des Kadavers eingesetzt werden.

### Stand der Technik

Neben der Erdbestattung und der Feuerbestattung ist die alkalische Hydrolyse als weitere Bestattungsmethode bekannt. Dabei wird organisches Material mittels einer starken Lauge hydrolysiert und somit zersetzt. Das organische Material kann ein Kadaver eines Tieres oder ein Leichnam eines Menschen sein. Hierfür ist ein Behälter vorgesehen, in den das organische Material eingelegt wird. Dann wird eine Base oder eine Lauge in den Behälter gegeben und Wasser zugeführt. Die Base bildet in der wässrigen Lösung eine Lauge. Die Lauge und das Wasser werden als Prozesswasser bezeichnet und dem organischen Material zugeführt. Herkömmlicherweise wird das Prozesswasser erhitzt und der Behälter steht unter Druck. Der Zersetzungsprozess kann dann abhängig vom Gewicht des organischen Materials, der Temperatur und dem Druck sowie der nachfolgend beschriebenen Agitation ungefähr 3 bis 20 Stunden in Anspruch nehmen. Nach der Zersetzung bleiben eine hier als Prozessabwasser bezeichnete Flüssigkeit sowie feste Knochenreste übrig. Das Prozessabwasser enthält die Lauge, Wasser sowie die zersetzen Überreste des organischen Materials. Sie ist mikrobiologisch steril und enthält keine DNS mehr. Schließlich wird das Prozessabwasser nachbehandelt. Dabei wird zum einen die Lauge neutralisiert, indem z. B. eine Säure hinzugegeben wird, und zum anderen das Prozessabwasser abgekühlt, indem typischerweise Frischwasser zugeführt wird. Schließlich kann das Prozessabwasser entsorgt werden.

Die Effektivität der Hydrolyse hängt von der Agitation ab. Unter Agitation versteht die vorliegende Anmeldung die Bewegung eines oder mehrerer Bestandteile eines Gemisches oder einer Flüssigkeit, um den Stoffkontakt, hier also den Kontakt mit dem Leichnam bzw. dem Kadaver, zu verbessern. Insbesondere ist damit das Umrühren oder anderweitige Bewegen der Flüssigkeit im Hinblick auf die damit zusammenhängende Wirkung der Beeinflussung bzw. der Effektivität des Stoffkontakts gemeint.

Die US 2013/0053615 A1 offenbart ein Verfahren und eine Vorrichtung zur alkalischen Hydrolyse von Gewebe, wie z. B. Leichnamen und/oder Kadavern bei Niedrigtemperatur. Die Vorrichtung weist einen waagerecht aufgestellten Behälter mit einem Deckel auf, in dem die Auflöseflüssigkeit gehalten wird. Es sind Beschickungskörbe zum Einbringen der Leichname in den Behälter vorgesehen. Das Verfahren wird durch eine Steuereinrichtung gesteuert. Zudem werden eine Pumpe, ein Abwasseranschluss, Ventile für Heiß-/Kaltwasser und eine Heizung beschrieben. Das Prozessabwasser kann optional mittels CO₂ neutralisiert werden.

Die WO 2011/137351 A2 offenbart ebenfalls ein Verfahren und eine Vorrichtung zur alkalischen Hydrolyse von Gewebe, wie z. B. Leichnamen und/oder Kadavern. Dabei ist ein Behandlungskessel vorgesehen, in den das Gewebe, also der Leichnam oder der Kadaver, in einem Korb eingelegt wird. Der Behandlungskessel wird mit Wasser und Lauge befüllt und die Prozessflüssigkeit verbleibt während des gesamten Prozesses in dem Behälter. Bei Angehörigen entsteht dadurch der Eindruck, dass der Verstorbene "ertränkt" wird. Der Behandlungskessel wird für den Prozess in Richtung der Vertikalen gekippt und zwischen 10 und 90° schräg gestellt. Dadurch rutschen die Überreste durch die Schwerkraft im Laufe des Prozesses zum unteren Ende des Korbs hinunter und können dort gesammelt werden. Die Agitation wird durch einen Quirl an der Unterseite des Behandlungskessels erzeugt. Aufgrund der höheren Agitation wird eine höhere Effektivität der Hydrolyse erzeugt als bei herkömmlichen Vorrichtungen, bei denen das Gewebe nur eingelegt wird. Allerdings ist die Wirkung der Agitation nicht gleichmäßig über den Behandlungskessel verteilt und am Boden höher. Da die Tür und Dichtungen über dem Level der Flüssigkeit liegen, werden diese Komponenten geschont und es erübrigt sich eine Wartung bzw. eine Instandhaltung. Optional kann der Kessel unter Druck stehen. Im Zusammenhang mit Bestattungen ist ein Schrägstellen oder Kippen des Behälters allerdings unerwünscht, da dies die Würde des Verstorbenen verletzt und von Angehörigen als pietätlos empfunden wird. Es wird hierfür auf den Code of Ethics der International Cremation Federation (ICF), Punkt 7 verwiesen, der besagt, dass ein Leichnam, sobald er in der Anlage ist, nicht mehr berührt oder beeinträchtigt werden darf.

Die US 2011/0130612 A1 offenbart eine alkalische Hydrolyseeinheit und ein Verfahren zu ihrer Verwendung zur Entsorgung einer Leiche, wobei die alkalische Hydrolyseeinheit Folgendes umfasst: eine Kammer zur Aufnahme einer chemisch zu zersetzenden Leiche, wobei die Kammer einen Kopfaufnahmeteil umfasst, der zur Aufnahme des Kopfes einer Leiche vorgesehen ist; eine Umwälzpumpe, die flüssigkeitsmäßig mit der Kammer der alkalischen Hydrolyseeinheit verbunden und zur Umwälzung von Flüssigkeiten innerhalb der Kammer geeignet ist; ein in der Kammer anordenbares Kopfhaltemittel zum Festhalten des Kopfes der Leiche im Kopfaufnahmeteil der Kammer; und mindestens eine Umwälzdüse, die flüssigkeitsmäßig mit der Umwälzpumpe verbunden und so positioniert ist, dass sie Flüssigkeit von der Umwälzpumpe auf den Kopfaufnahmeteil der Kammer richtet.

Es ist die Aufgabe der vorliegenden Erfindung, eine Vorrichtung und/oder ein Verfahren bereitzustellen, das eine höhere und gleichmäßig verteilte Agitation erreicht, um die Effektivität der Hydrolyse zu erhöhen, den Ressourcenverbrauch, den Spülaufwand und den Pflegeaufwand zu verringern, und bei dem/der zeitgleich die Würde des Verstorbenen und die Pietät gegenüber den Angehörigen gewahrt wird.

### Offenbarung der Erfindung

Die Aufgabe wird gelöst durch eine Vorrichtung zur alkalischen Hydrolyse von Leichnamen und/oder Kadavern, welche Düsen zur Verteilung eines Prozesswassers aufweist. Die Vorrichtung weist eine Prozesswanne auf, in die der Leichnam oder der Kadaver eingebracht wird. Hierfür kann der Leichnam bzw. Kadaver direkt in die Prozesswanne und/oder zuvor in einen weiter unten beschriebenen Beschickungskorb eingelegt werden. Dabei werden, soweit möglich, Fremdkörper entfernt. Bei Leichnamen werden beispielsweise Kleidung, Dreingaben, Verpackung und Ähnliches im Vorhinein entfernt. Eine Haube umschließt die Prozesswanne während der Behandlung. Die Haube kann auf einer Seite durch eine Tür abgeschlossen werden und auf der anderen Seite eine verschließbare Öffnung aufweisen.

Die Vorrichtung weist einen Präparationsbehälter auf, in dem das Prozesswasser zur alkalischen Hydrolyse vorbereitet wird. Hierfür wird eine Lauge und/oder eine Base, die in wässriger Lösung die Lauge bildet, in den Behandlungsbehälter eingebracht. Als Beispiel kann eine Kalilauge als Lauge eingebracht werden. Bevorzugt kann die Base Kaliumhydroxid sein, die direkt eingebracht wird und in wässriger Lösung Kalilauge im Behälter bildet. Kaliumhydroxid kann als Feststoff in den Behälter eingebracht werden, was bezüglich des Platzbedarfs von Vorteil ist. Alternativ kann auch Natronlauge bzw. Natriumhydroxid oder andere Laugen bzw. Basen verwendet werden. Es kann ein Dosierbehälter vorgesehen sein, in dem die Lauge bzw. die Base vorgehalten wird. Der Dosierbehälter dosiert dann die Lauge bzw. die Base mengengenau in den Präparationsbehälter ein. Während des Betriebs kann über den Dosierbehälter weiter Lauge bzw. Base zugeführt werden, um den gewünschten pH-Wert von vorzugsweise über 12 zu halten.

Ferner ist zumindest ein Frischwasserzulauf an dem Präparationsbehälter vorgesehen, über den eine vorgebbare Menge Frischwasser in den Präparationsbehälter zugeführt werden kann. Die Menge an Frischwasser, die dem Präparationsbehälter zugeführt wird, ist entweder die Menge für eine komplette Füllung des Präparationsbehälters oder die Menge, die aus der Vorrichtung abgeführt wird und/oder wie unten beschrieben, in den Konditionierungsbehälter umgepumpt wird. Der zumindest eine Frischwasserzulauf kann zudem mit anderen Komponenten der Vorrichtung verbunden sein, wodurch Frischwasser zur Reinigung der jeweiligen Komponente verwendet werden kann.

Es ist zumindest eine Düse in der Haube vorgesehen. Alternativ oder zusätzlich ist zumindest eine Düse in der Prozesswanne vorgesehen. Die zumindest eine Düse ist während der Behandlung über Leitungen mit dem Präparationsbehälter verbunden. Das Prozesswasser wird von dem Präparationsbehälter durch die Leitungen zu der zumindest einen Düse geführt und mittels der zumindest einen Düse über den Leichnam bzw. den Kadaver in der Prozesswanne gesprüht. Durch die zumindest eine Düse wird das Prozesswasser über den Leichnam bzw. den Kadaver verteilt und die Lauge kommt in direkten Kontakt mit dessen Oberfläche. Eine Düse in der Haube besprüht den Leichnam bzw. den Kadaver von oben und gegebenenfalls von der Seite. Eine Düse in der Prozesswanne ist vorzugsweise so angeordnet, dass sie den Leichnam bzw. den Kadaver von unten und/oder von der Seite besprüht. Generell ist bereits eine Düse entweder in der Haube oder in der Prozesswanne ausreichend, um den Leichnam bzw. den Kadaver zu besprühen. Vorzugsweise sind mehrere Düsen vorgesehen, wodurch das Prozesswasser gezielter verteilt werden kann und somit die Zersetzung schneller erfolgt. Dabei ist die Kombination von Düsen in der Haube und Düsen in der Prozesswanne am besten geeignet, um den Leichnam bzw. den Kadaver aus allen Richtungen zu besprühen.

Durch das Besprühen mit dem Prozesswasser findet die alkalische Hydrolyse statt, bei der der Leichnam bzw. der Kadaver durch die Lauge zersetzt wird. Die Düsen bewirken durch das Besprühen eine bessere und kontinuierliche Verteilung des Prozesswassers auf der gesamten Oberfläche des Leichnams bzw. des Kadavers. Als Resultat wird eine gleichmäßig hohe Agitation erreicht. Wenn für einen speziellen Prozessschritt in der Prozesswanne ein Bad erzeugt werden soll, bewirken die Düsen aufgrund des Wasserstroms, der mit einer Rührfunktion vergleichbar ist, eine Durchmischung des Bads, was ebenfalls zu einer höheren Agitation führt. Des Weiteren wird die Vorrichtung nicht schräg gestellt oder gekippt, sondern bleibt ebenerdig stehen und horizontal ausgerichtet. Wird, wie bei anderen Vorrichtungen üblich, ein Behandlungsbehälter schräg gestellt, erweckt dies bei einem Beobachter den problematischen Eindruck, dass der Leichnam bzw. der Kadaver "ertränkt" wird. Die erfindungsgemäße Vorrichtung wird nicht schräg gestellt oder gekippt, sodass der Leichnam bzw. Kadaver in der Horizontalen verbleibt. Zudem arbeitet die Vorrichtung bei Atmosphärendruck. Wird, wie bei anderen Vorrichtungen üblich, ein hoher Druck angelegt, um das Prozesswasser mit Temperaturen von über 100°C zu betreiben, erweckt dies bei einem Beobachter den problematischen Eindruck, dass der Leichnam bzw. der Kadaver gekocht wird. Die erfindungsgemäße Vorrichtung arbeitet bei Temperaturen unter 100°C. Insgesamt wird mit der erfindungsgemäßen Vorrichtung die Würde des Verstorbenen und die Pietät gegenüber den Angehörigen gewahrt.

Der Zersetzungsprozess kann - je nach Gewicht des Leichnams bzw. des Kadavers, der Temperatur und dem Druck sowie der Agitation - ca. 3 bis 20 Stunden dauern. Der Zersetzungsprozess läuft bei atmosphärischen Druck ab, es wird also kein Überdruck benötigt.

Überreste, wie z. B. Knochen, verbleiben in der Prozesswanne und können schließlich separat entnommen werden. Es kann optional vorgesehen sein, die Knochen zu zerkleinern, insbesondere zu zermahlen, und sie dann an einen Bestatter, einen Angehörigen oder, im Falle eines Tieres, an dessen Besitzer auszuhändigen.

Zudem ist ein Abwasserabfluss vorgesehen, über den das Prozessabwasser, welches vorher vorzugsweise neutralisiert wird, in ein Abwassersystem eingeleitet wird.

Zum Transport des Prozesswassers können Leitungen und Ventile vorgesehen sein. Die Ventile können teilweise als 3-Wege-Ventile ausgebildet sein. Als "Umschalten" eines Ventils versteht die vorliegende Anmeldung, dass die Stellung des Ventils so geändert wird, dass die Flüssigkeit durch eine andere Leitung geführt wird. Es ist zumindest eine Pumpe vorgesehen, welche das Prozesswasser vom Präparationsbehälter durch die Leitungen zu der zumindest einen Düse pumpt. Das ablaufende Prozesswasser kann bei der Behandlung durch die Pumpe kontinuierlich erneut den Düsen zugeführt werden und somit auf den Leichnam bzw. Kadaver gesprüht werden. Dadurch wird sichergestellt, dass genügend Prozesswasser den Leichnam bzw. den Kadaver erreicht.

Es ist eine speicherprogrammierbare Steuerung vorgesehen, welche die Vorrichtung steuert. Zusätzlich können über eine Schnittstelle dem Benutzer Prozessschritte und auszuführende Aktionen angezeigt werden. Außerdem können über die gleiche Schnittstelle oder über eine weitere Schnittstelle Anfragen der Steuerung vom Benutzer beantwortet werden und Werte und/oder Parameter eingegeben werden.

Unterhalb der Prozesswanne ist eine Auffangwanne angeordnet, welche das Prozesswasser aus der Prozesswanne und das Prozesswasser, das von der zumindest einen Düse ausgesprüht wird und in Kontakt mit dem Leichnam bzw. dem Kadaver getreten ist, auffängt. Die Prozesswanne muss daher nicht vollkommen dicht sein, da das Prozesswasser von der Auffangwanne aufgefangen wird. Die Prozesswanne weist bevorzugt einen Auslass auf, über den das Prozesswasser in die Auffangwanne abgeleitet wird. Vorzugsweise ist der Auslass als Schieber ausgebildet, der von außen geöffnet und geschlossen werden kann. Die Prozesswanne, in die der Leichnam bzw. der Kadaver eingelegt wird, und die Auffangwanne, welche das Prozesswasser auffängt, sind separat voneinander ausgebildet. Die Auffangwanne ist mit dem Präparationsbehälter verbunden, sodass das Prozesswasser aus der Auffangwanne in den Präparationsbehälter rückgeführt wird und von dort erneut den Düsen zugeführt werden kann. Die Auffangwanne kann zumindest einen Ablauf aufweisen, durch den das Prozesswasser abgeführt werden kann. Optional ist die Auffangwanne mit einem weiter unten näher beschriebenen Konditionierungsbehälter, den die Vorrichtung vorteilhafterweise aufweist, verbunden.

Die Prozesswanne ist zu der Auffangwanne beweglich, insbesondere verschiebbar, gelagert, beispielsweise durch Rollen an der Unterseite der Prozesswanne. Die Prozesswanne kann in eine Behandlungsposition geschoben werden, in der die Prozesswanne über einem Behandlungsabschnitt der Auffangwanne angeordnet ist. Der Behandlungsabschnitt der Auffangwanne kann einen Ablauf aufweisen, der über Leitungen mit dem Präparationsbehälter verbunden ist. Somit kann während der Behandlung das Prozesswasser aus der Auffangwanne über den Ablauf im Behandlungsabschnitt zu dem Präparationsbehälter geleitet werden und von dort kontinuierlich erneut der zumindest einen Düse zugeführt werden. Vorteilhafterweise ist der Ablauf über weitere Leitungen mit dem Konditionierungsbehälter verbunden. Mit zumindest einem Ventil in den Leitungen kann die Verbindung des Behandlungsabschnitts zum Präparationsbehälter oder zum Konditionierungsbehälter gewählt werden. Wenn der Zersetzungsprozess beendet ist, kann das zumindest eine Ventil umgeschaltet werden und das Prozessabwasser und/oder Wasser, das bei der Reinigung anfällt, aus der Auffangwanne über bevorzugt denselben Ablauf im Behandlungsabschnitt zu dem Konditionierungsbehälter geleitet werden. Rein prinzipiell können auch mehrere Abläufe im Behandlungsabschnitt der Auffangwanne vorgesehen sein, die mit dem Präparationsbehälter bzw. dem Konditionierungsbehälter verbunden sind und anstelle des Ventils entsprechend geöffnet und geschlossen werden.

Des Weiteren kann die Prozesswanne in eine Reinigungsposition geschoben werden, in der die Prozesswanne über einem Reinigungsabschnitt der Auffangwanne angeordnet ist. Der Reinigungsabschnitt der Auffangwanne kann einen Ablauf aufweisen, der über Leitungen mit dem Abwasserabfluss verbunden ist, wobei vorzugsweise eine Nachbehandlung im nachfolgend beschriebenen Konditionierungsbehälter zwischengeschaltet ist. Vorzugsweise bewegt sich die Haube nicht mit in die Reinigungsposition. In der Reinigungsposition der Prozesswanne können die Prozesswanne und die darin befindlichen Überreste, wie z. B. Knochen, mit Frischwasser gespült werden. Ferner ist die von der Prozesswanne getrennte Haube besser für die Reinigung zugänglich. Zumindest ein Teil der Reinigung kann auch in der Behandlungsposition der Prozesswanne durchgeführt werden. So können beispielsweise die zumindest eine Düse und die Leitungen in der Behandlungsposition der Prozesswanne gespült werden. Die Überreste können in der Reinigungsposition einfach entnommen werden. Zudem können die Überreste räumlich getrennt von der Position, in der der Leichnam bzw. der Kadaver eingelegt wird, entnommen werden. Dadurch kann die Pietät gegenüber den Angehörigen gewahrt werden, die den Entnahmeprozess nicht miterleben. Dies gilt insbesondere, wenn die Haube in der Behandlungsposition verbleibt und ein Sichtschutz, beispielsweise durch eine Wand, außerhalb der Vorrichtung vorgesehen ist.

Der vorstehend erwähnte Konditionierungsbehälter dient als Neutralisierungskomponente für das Prozessabwasser dient. Der Konditionierungsbehälter ist über zumindest eine Leitung mit dem Präparationsbehälter verbunden. Das Prozessabwasser wird über die zumindest eine Leitung vom Präparationsbehälter zum Konditionierungsbehälter geführt. Hierfür kann die zumindest eine Pumpe verwendet werden. Wasser, das bei der Reinigung verwendet wurde, wird ebenfalls über die genannten Leitungen zum Konditionierungsbehälter geführt.

Im Konditionierungsbehälter ist eine Einrichtung zur Neutralisation der Lauge im Prozessabwasser vorhanden. Damit wird im Rahmen einer Konditionierung mittels eines Neutralisierungsmittels eine Neutralisation der Lauge und somit des Prozessabwassers im Konditionierungsbehälter durchgeführt. Das Neutralisierungsmittel ist vorzugsweise eine Säure, insbesondere Zitronensäure. Der Konditionierungsbehälter ist zudem mit dem Abwasserabfluss verbunden, über den das neutralisierte Prozessabwasser aus dem Konditionierungsbehälter in ein Abwassersystem abgeführt werden kann.

Die Einrichtung zur Neutralisation der Lauge ist vorzugsweise eine Dosiereinrichtung, die eingerichtet ist, die flüssige oder feste Säure in den Konditionierungsbehälter einzuleiten. Vorzugsweise wird eine flüssige Säure zur Neutralisation der Lauge im Prozessabwasser verwendet. Bevorzugt wird eine Zitronensäure und besonders bevorzugt eine 50%-ige Zitronensäure verwendet. Die Einrichtung zur Neutralisierung der Lauge kann in diesem Fall eine Dosierpumpe sein, die eingerichtet ist, die flüssige Säure in den Konditionierungsbehälter einzuleiten. Dies bietet den Vorteil, dass eine benötigte Menge der Säure zur Neutralisierung der Lauge passend genau eingeleitet werden kann.

Mit einer entsprechenden Steuerung der Ventile ist eine Pumpe in der Vorrichtung ausreichend, um sowohl das Prozesswasser zu der zumindest einen Düse zu pumpen als auch das Prozessabwasser in den Konditionierungsbehälter zu pumpen. Vorzugsweise weist die Vorrichtung zwei Pumpen auf. Eine Pumpe dient zur Förderung des Prozesswassers vom Präparationsbehälter zu der zumindest einen Düse. Nach dem Besprühen läuft das Prozesswasser durch entsprechend gesteuerte Ventile zurück in den Präparationstank. Der Präparationstank und die entsprechenden Leitungen sind vorzugsweise unterhalb der Prozesswanne angeordnet, sodass das Prozesswasser aufgrund von Schwerkraft in den Präparationstank zurückläuft. Zudem dient diese Pumpe zur Förderung des Prozessabwassers vom Präparationstank in den Konditionierungsbehälter. Eine weitere Pumpe dient der Zirkulation während der Neutralisierung um den Konditionierungsbehälter und zur Förderung des Prozessabwassers in den Abwasserabfluss Somit kann die Zirkulation des Prozesswassers zu der zumindest einen Düse und die Zirkulation des Prozessabwassers um den Konditionierungsbehälter unabhängig voneinander durchgeführt werden.

Vorteilhafterweise besteht eine Verbindung zwischen den beiden Leitungssystemen, die über ein Ventil getrennt werden kann. Im normalen Betrieb ist das Ventil geschlossen und die Verbindung getrennt, sodass die Pumpen getrennt die Zufuhr des Prozesswassers und die Abfuhr des Prozessabwassers durchführen können. Fällt eine der Pumpen aus, so kann im Notbetrieb die Verbindung geöffnet werden und die andere, funktionierende Pumpe kann sowohl die Förderung und Zirkulation des Prozesswassers vom Präparationsbehälter zu der zumindest einen Düse als auch die Förderung und Zirkulation des Prozessabwassers in den Konditionierungsbehälter und zum Abwasserabfluss ausführen.

Bevorzugt ist die Haube zu der Prozesswanne beweglich, insbesondere verschiebbar, gelagert, beispielsweise durch Rollen an der Unterseite der Haube. Die Haube kann in eine Einlegeposition geschoben werden, in der die Haube von der Prozesswanne entfernt ist und somit die Prozesswanne von außen frei zugänglich ist. In dieser Einlegeposition kann der Leichnam bzw. der Kadaver einfach in die Prozesswanne eingelegt werden. Des Weiteren kann die Haube in eine Behandlungsposition geschoben werden, in der die Haube die Prozesswanne umschließt. In dieser Behandlungsposition findet die alkalische Hydrolyse statt und das Prozesswasser wird durch die Düsen auf den Leichnam bzw. den Kadaver gesprüht.

Vorteilhafterweise sind die Prozesswanne und die Haube unabhängig voneinander beweglich, insbesondere verschiebbar, gelagert, sodass diese ohne Behinderung zueinander bewegt, insbesondere verschoben, werden können. Bevorzugt entsprechen sich die Behandlungsposition der Prozesswanne, in der die Prozesswanne über dem Behandlungsabschnitt der Auffangwanne angeordnet ist, und die Behandlungsposition der Haube, in der die Haube über der Prozesswanne angeordnet ist, sodass sich im Ergebnis die Haube während der Behandlung über dem Behandlungsabschnitt der Auffangwanne befindet und das Prozesswasser von der zumindest einen Düse in der Haube optimal im Behandlungsabschnitt der Auffangwanne aufgefangen wird. Die Haube kann zudem für die Reinigung in die Einlegeposition verschoben werden. Insbesondere, wenn die Prozesswanne ebenfalls in die Reinigungsposition geschoben wurde, kann der Behandlungsabschnitt der Auffangwanne von außen gereinigt werden. Indem die Prozesswanne und/oder die Haube zur Reinigung verschoben werden können, wird eine einfache Reinigung erreicht. Außerdem muss die erfindungsgemäße Vorrichtung nicht wie bei herkömmlichen Vorrichtungen zur Reinigung oder zur Entnahme der Überreste schräg gestellt oder gekippt werden. Dadurch werden die Würde des Verstorbenen und die Pietät gegenüber den Angehörigen gewahrt.

Die Verbindung zwischen der zumindest einen Düse in der Haube und/oder in der Prozesswanne und dem Präparationsbehälter kann bevorzugt lösbar ausgebildet sein. Hierfür ist bevorzugt zumindest eine lösbare Steckkupplung vorgesehen, über die die zumindest eine Düse in der Haube und/oder in der Prozesswanne mit dem Präparationsbehälter verbunden ist. Dies ist besonders vorteilhaft beim Bewegen, insbesondere Verschieben, der Prozesswanne und/oder der Haube, wie vorstehend beschrieben. So kann die Leitung zu der zumindest einen Düse in der Haube in der Einlegeposition getrennt werden und in der Behandlungsposition kann die Steckkupplung die Leitung zu der zumindest einen Düse in der Haube verbinden. Gleichermaßen kann die Leitung zu der zumindest einen Düse in der Prozesswanne in der Reinigungsposition getrennt werden und in der Behandlungsposition kann die Steckkupplung die Leitung zu der zumindest einen Düse in der Prozesswanne verbinden.

Vorteilhafterweise ist in zumindest einem Ablauf für das Prozesswasser ist ein mehrstufiger Filter angeordnet, der Gewebeteile und kleinste Schwebstoffe aus dem Prozesswasser filtert. Bei Verwendung der oben beschriebenen Auffangwanne, ist der Filter bevorzugt in einem Ablauf der Auffangwanne angeordnet, da hier das Prozesswasser zusammenläuft. Der mehrstufige Filter kann insbesondere drei Filterstufen aufweisen. Die erste Filterstufe stoppt größere Partikel und kleinere Knochen. Die zweite Filterstufe filtert kleinere Partikel heraus. Die dritte Filterstufe ist bevorzugt eine Filtereinheit aus einem Naturmaterial, wie z. B. Baumwolle, die Schwebstoffe und feine Partikel, die zum Leichnam bzw. Kadaver gehören, aufhält. Der mehrstufige Filter ist so ausgebildet, dass die Filtereinheit der dritten Filterstufe ausgewechselt werden kann. Die Filtereinheit der dritten Filterstufe ist vorzugsweise zur einmaligen Verwendung ausgebildet und kann für jeden Leichnam bzw. Kadaver separat eingesetzt werden. Zudem kann die Filtereinheit ein Kennzeichen, beispielsweise eine Nummer, aufweisen mit der die Filtereinheit dem Leichnam bzw. Kadaver nach der Hydrolyse zugeordnet werden kann. Das Filtergut sowie die Filtereinheit der dritten Stufe können getrocknet und zusammen mit den Überresten vermahlen werden und schließlich an einen Bestatter, einen Angehörigen oder, im Falle eines Tieres, an dessen Besitzer ausgehändigt werden. Nachdem das Prozesswasser den mehrstufigen Filter durchlaufen hat, sind darin keine Bestandteile des Körpers des Leichnams bzw. des Kadavers mehr enthalten. Es kommt folglich zu keiner Vermischung von Bestandteilen verschiedener Leichname bzw. Kadaver.

Kadaver und Leichname können mitunter ein hohes Gewicht aufweisen, wodurch es schwierig sein kann, diese in den Behandlungsbehälter einzubringen. Abhilfe bietet hierfür ein Beschickungskorb, der den Kadaver oder den Leichnam aufnimmt. Der Kadaver bzw. Leichnam kann mit dem Beschickungskorb zu der Prozesswanne transportiert werden. Hierfür kann eine Transportvorrichtung, wie z. B. ein Transportwagen, verwendet werden. Die Transportvorrichtung kann bevorzugt höhenverstellbar sein. Der Beschickungskorb wird dann in die Prozesswanne eingelegt. Der Beschickungskorb mit dem Leichnam bzw. Kadaver wird von der Transportvorrichtung durch die geöffnete Tür in die Prozesswanne geschoben und dort fixiert. Dadurch wird das Einbringen des Leichnams bzw. des Kadavers vereinfacht. Zudem ist der Beschickungskorb nicht komplett geschlossen, sondern ist zumindest teilweise wasserdurchlässig. Insbesondere weist der Beschickungskorb zumindest an den Stellen der Düsen eine durchlässige Struktur wie beispielsweise ein Gitter oder ein Gestänge auf. Durch den offenen Beschickungskorb wird sichergestellt, dass sich das Prozesswasser direkt auf die Oberfläche des Leichnams bzw. Kadavers verteilt, ohne durch den Beschickungskorb gehindert zu werden und an Wirkung zu verlieren.

Für Tierkadaver können auch mehrere Beschickungskörbe vorgesehen sein, wobei jeder Beschickungskorb beispielsweise einen einzelnen Kadaver aufnimmt. Bevorzugt können in die Prozesswanne mehrere Beschickungskörbe eingelegt werden, sodass mehrere Kadaver zeitgleich hydrolysiert werden können. Die mehreren Beschickungskörbe bieten zudem den Vorteil, dass die nach der Zersetzung übrigbleibenden festen Überreste, also insbesondere Knochen, nach Kadaver getrennt voneinander aus dem Beschickungskorb entnommen werden können. Rein prinzipiell können auch mehrere Kadaver in einen Beschickungskorb gelegt werden.

Im Leitungssystem des Präparationsbehälters kann ein Heizer vorgesehen sein, um das Prozesswasser während des Zersetzungsprozesses zu erwärmen. Durch die Erwärmung des Prozesswassers und der darin enthaltenen Lauge wird der Zersetzungsprozess beschleunigt. Ein bevorzugter Temperaturbereich des Prozesswassers während des Zersetzungsprozesses beträgt ca. 92 °C bis 98 °C.

Alternativ oder zusätzlich kann ein Wärmetauscher vorgesehen sein, mit dem Wärme aus dem Prozessabwasser vom Konditionierungsbehälter zum zumindest einen Präparationsbehälter transportiert wird, um dort das Prozesswasser zu erwärmen. Der Wärmetauscher entzieht dem Prozessabwasser im Konditionierungsbehälter die Wärme, sodass es schneller abkühlt und somit auch früher abgeführt werden kann, und führt die Wärme direkt dem Prozesswasser im zumindest einen Präparationsbehälter zu, sodass sich dieses erwärmt. Wie oben beschrieben, wird durch die Erwärmung des Prozesswassers der Zersetzungsprozess beschleunigt. Zudem wird durch den Wärmetauscher der Energiebedarf gesenkt.

Die Vorrichtung umfasst vorzugsweise zumindest eine Messeinrichtung für eine oder mehrere der folgenden Parameter:
- Durchflussmenge des Prozesswassers zu der zumindest einen Düse;
- Temperatur des Prozesswassers;
- pH-Wert des Prozesswassers;
- Menge der zugeführten Base bzw. Lauge;
- zugeführte Wassermenge;
- Temperatur des Prozessabwassers;
- pH-Wert des Prozessabwassers;
- Leitfähigkeit des Prozessabwassers.

Die Temperatur des Prozesswassers wird ermittelt, um diese auf den optimalen Temperaturbereich für den Zersetzungsprozess einzuregeln, der bevorzugt bei ca. 92 °C bis 98 °C liegt. Der pH-Wert des Prozesswassers wird ermittelt, um bei Bedarf weitere Lauge oder Base aus dem Dosierbehälter in den Präparationsbehälter zu dosieren. Die Menge der zugeführten Base bzw. Lauge und des Wassers werden ermittelt, um diese mit den Vorgaben entsprechend der Masse des Leichnams bzw. Kadavers abzugleichen. Die Temperatur des Prozessabwassers und der pH-Wert des Prozessabwassers werden ermittelt, um festzustellen, ob und wann das Prozessabwassers in das Abwassersystem entlassen werden kann. Anstelle der direkten Messung des pH-Werts kann auch die Leitfähigkeit des Prozessabwassers gemessen werden.

Optional kann eine oder mehrere der folgenden Sicherheitseinrichtungen vorgesehen sein:
- Sicherheitsdruckventil im Präparationsbehälter und/oder im zumindest einen Konditionierungsbehälter;
- Sicherheitstemperaturbegrenzer im Präparationsbehälter;
- Leckagewanne für Leckageflüssigkeit, die elektronisch überwacht wird.

Der Präparationsbehälter, die Haube, die Prozesswanne, die Auffangwanne und/oder der Konditionierungsbehälter können vorzugsweise aus Edelstahl oder Kunststoff gefertigt sein, die nicht von der alkalischen Hydrolyse zersetzt werden. Die Materialauswahl ist allerdings nicht darauf beschränkt und es können auch andere Materialien mit diesen Eigenschaften verwendet werden. Der Präparationsbehälter und/oder der Konditionierungsbehälter können zudem als verschlossene Tanks ausgebildet sein.

Die Aufgabe wird des Weiteren durch ein Verfahren zur alkalischen Hydrolyse von Leichnamen und/oder Kadavern mit den folgenden Schritten gelöst.

In den Präparationsbehälter wird eine berechnete Wassermenge durch einen Frischwasserzulauf zugeführt. Die Menge an Frischwasser, die dem Präparationsbehälter zugeführt wird, ist entweder die Menge für eine komplette Füllung des Präparationsbehälters oder die Menge, die aus der Vorrichtung abgeführt wird und/oder, wie unten beschrieben, in den Konditionierungsbehälter umgepumpt wird.

Im Anschluss wird eine Lauge und/oder eine Base, die in wässriger Lösung die Lauge bildet, in den Präparationsbehälter eingebracht. Die Lauge kann z. B. Kalilauge sein und die Base kann z. B. Kaliumhydroxid, das in wässriger Lösung Kalilauge bildet, sein. Kaliumhydroxid kann als Feststoff in den Behälter eingebracht werden, was bezüglich des Platzbedarfs von Vorteil ist. Alternativ können auch Natronlauge bzw. Natriumhydroxid oder andere Laugen bzw. Basen verwendet werden. Die eingebrachte Menge der Lauge bzw. Base wird abhängig vom pH-Wert des Prozesswassers. Dabei wird das Prozesswasser vorzugsweise auf einen pH-Wert von über 12 gebracht. Die Lauge bzw. Base ist in einem Dosierbehälter vorgelagert und wird mengengenau in den Präparationsbehälter eindosiert.

Der Leichnam oder der Kadaver wird in die Prozesswanne eingelegt. Hierfür befindet sich die Prozesswanne vorzugsweise in der Behandlungsposition, rein prinzipiell kann sie aber auch einer anderen für das Einlegen geeigneten Position befinden. Die Haube befindet sich vorzugsweise in der Einlegeposition, sodass die Prozesswanne von außen frei zugänglich ist. Vor dem Einlegen werden, soweit möglich, Fremdkörper entfernt. Bei Leichnamen werden beispielsweise Kleidung, Dreingaben, Verpackung und Ähnliches im Vorhinein aussortiert.

Zur Einbringung des Kadavers oder des Leichnams kann dieser in einen Beschickungskorb gelegt werden und der Beschickungskorb in die Prozesswanne eingelegt werden. Im Fall von Tierkadavern können auch mehrere Beschickungskörbe vorgesehen sein. Zur Einbringung kann die oben beschriebene Transportvorrichtung verwendet werden.

Nachdem der Leichnam bzw. der Kadaver in die Prozesswanne eingelegt wurde, wird die Haube - falls sie sich dort nicht bereits befindet - in eine Behandlungsposition, in der sich die Haube über der Prozesswanne befindet, verschoben.

Bevorzugt wird das Prozesswasser wie vorstehend beschrieben bereits zu Beginn im Präparationsbehälter vorbereitet. Es kann aber auch vorgesehen sein, das Prozesswasser erst nach dem Einlegen des Leichnams bzw. des Kadavers oder nachdem die Haube in die Behandlungsposition gebracht wurde vorzubereiten.

Das Prozesswasser wird nun vom Präparationsbehälter zu der zumindest einen Düse in der Haube und/oder in der Prozesswanne geführt und auf den Leichnam bzw. Kadaver gesprüht. Dadurch findet der Zersetzungsprozess statt. Das Prozesswasser, das in Kontakt mit dem Leichnam bzw. dem Kadaver getreten ist, wird in den Präparationstank zurückgeführt, vorzugsweise unter Ausnutzung der Schwerkraft. Vorzugsweise wird das Prozesswasser zuvor von einer Auffangwanne aufgefangen. Dann wird das Prozesswasser durch Pumpen wiederholt der zumindest einen Düse zugeführt. Das Besprühen wird für eine vorgegebene Prozesszeit, die - je nach Masse Leichnams oder Kadavers, der Temperatur und dem Druck sowie der Agitation - ca. 3 bis 20 Stunden dauert, fortgesetzt. Wenn für einen speziellen Prozessschritt in der Prozesswanne ein Bad erzeugt werden soll, wird zwischenzeitlich mehr Prozesswasser durch die Düsen ausgesprüht als zeitgleich in den Präparationstank zurückgeführt wird. Zudem wird das Prozesswasser geheizt. Hierfür kann die oben beschriebene Heizung und/oder der oben beschriebene Wärmetauscher verwendet werden.

Nach Ende der Zersetzung und bevorzugt auch bereits während der Zersetzung kann Prozessabwasser aus dem Präparationstank in den Konditionierungsbehälter überführt und dort neutralisiert werden. Hierbei wird aus dem Präparationstank nur ein Teil der Flüssigkeit, beispielsweise ein Drittel des gesamten Flüssigkeit im Präparationstank entnommen. Zum Aufbereiten des Prozessabwassers wird eine Menge eines Neutralisierungsmittels, insbesondere einer Säure in den Konditionierungsbehälter hinzugegeben, bis der pH-Wert des Prozessabwassers einen Schwellenwert für den pH-Wert unterschritten hat. Der pH-Wert des Prozessabwassers wird vorzugsweise kontinuierlich gemessen. Der Schwellenwert für den pH-Wert wird so gewählt, dass das Prozessabwasser gefahrlos durch den Abwasserabfluss in das Abwassersystem entlassen werden kann, und liegt beispielsweise bei 9,5. Unterschreitet der pH-Wert den Schwellenwert, so wird die Zugabe des Neutralisierungsmittels gestoppt.

Optional kann abgewartet werden, bis das Prozessabwasser unter einen Temperaturschwellenwert abgekühlt ist. Die Temperatur des Prozessabwassers wird vorzugsweise ebenfalls kontinuierlich gemessen. Der Temperatur-schwellenwert wird so gewählt, dass das Prozessabwasser gefahrlos durch den Abwasserabfluss in das Abwassersystem entlassen werden kann, und liegt beispielsweise bei 40°C.

Schließlich wird das Prozessabwasser durch den Abwasserabfluss abgepumpt und in das Abwassersystem abgeführt.

Nach Ende des Zersetzungsprozesses kann die Prozesswanne in eine Reinigungsposition verschoben werden. Anschließend werden die Prozesswanne und die Haube sowie gegebenenfalls die Auffangwanne gereinigt. Dies kann beispielsweise durch einen Benutzer von Hand durchgeführt werden, der hierfür Frischwasser aus einem Frischwasserzulauf verwenden kann. Da die Haube und die Prozesswanne unabhängig voneinander beweglich, insbesondere verschiebbar, sind, kann die Reinigung in einfacher Weise in der Horizontalen durchgeführt werden. Schließlich wird das Abwasser abgeführt.

Es kann zudem vorgesehen sein, den Präparationsbehälter und gegebenenfalls den Konditionierungsbehälter vollständig zu reinigen. Hierfür können der Präparationsbehälter, gegebenenfalls der Konditionierungsbehälter und die damit verbundenen Leitungen mit Frischwasser aus dem Frischwasserzulauf gespült werden. Die Düsen können bereits vor der Reinigung der Prozesswanne bzw. der Auffangwanne oder zu diesem Zeitpunkt gespült werden. Zur Förderung des Spülwassers kann ebenfalls die zumindest eine Pumpe verwendet werden. Schließlich wird das Spülwasser aus den genannten Komponenten in den Konditionierungsbehälter abgeleitet. Während des Spülvorgangs kann auch der Konditionierungsbehälter gespült werden.

Das beim Reinigen anfallende Abwasser/Spülwasser kann dem Konditionierungsbehälter zugeführt werden und dort gleichermaßen neutralisiert werden. Nach der Nachbereitung kann es zusammen mit dem Prozessabwasser durch den Abwasserabfluss abpumpt werden. Alternativ kann es zeitlich versetzt, insbesondere nach dem Prozessabwasser durch den Abwasserabfluss abgepumpt werden.

Das vorstehend beschriebene Verfahren wird vorzugsweise durch die obengenannte speicherprogrammierbare Steuerung ausgeführt.

Im Ergebnis wird durch regelmäßiges Zuführen von Frischwasser, die Wassermenge in der Vorrichtung konstant gehalten und durch regelmäßiges Zuführen von Lauge bzw. Base während des Prozesses der pH-Wert über einem gewünschten Wert, der beispielsweise 12 beträgt, gehalten. Dadurch wird sichergestellt, dass die nur die erforderliche Menge Lauge bzw. Base verwendet wird. Das Prozesswasser wird laufend wieder aufbereitet, ohne dass das komplette Prozesswasser am Ende jedes Prozesses abgelassen werden muss. Dies bietet den Vorteil, dass insgesamt weniger Wasser, weniger Lauge bzw. Base und weniger Wärme bzw. Energie benötigt wird. Da das Kühlen und Neutralisieren während des Prozesses stattfinden, wird außerdem die gesamte Prozessdauer verringert.

### Kurze Beschreibung der Zeichnungen

Ausführungsbeispiele der Erfindung sind in den Zeichnungen dargestellt und in der nachfolgenden Beschreibung näher erläutert.
- Figur 1: zeigt eine Außenansicht der erfindungsgemäßen Vorrichtung zur alkalischen Hydrolyse von Leichnamen und/oder Kadavern.
- Figur 2: zeigt eine schematische Darstellung der erfindungsgemäßen Vorrichtung.
- Figur 3: zeigt ein Ablaufdiagramm des erfindungsgemäßen Verfahrens zur alkalischen Hydrolyse von Leichnamen und/oder Kadavern.
- Figuren 4 bis 12: zeigen die Vorrichtung aus Figur 2 in verschiedenen Etappen der alkalischen Hydrolyse mit den jeweils verwendeten Leitungssystemen.
- Figur 13: zeigt die Vorrichtung aus Figur 2 während eines Notbetriebs.

### Ausführungsformen der Erfindung

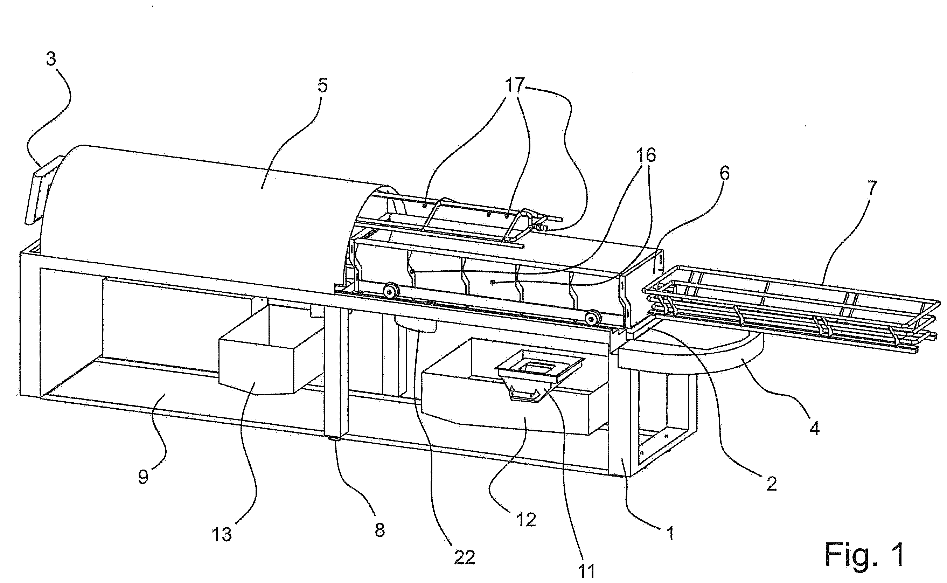

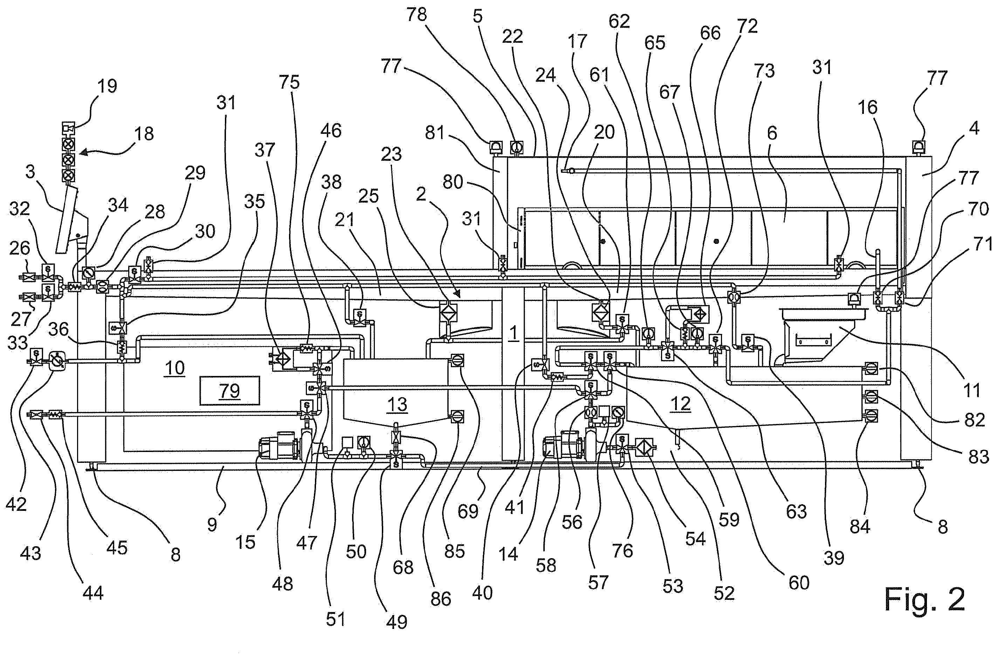

Figur 1 zeigt eine Außenansicht der erfindungsgemäßen Vorrichtung mit einem Gehäuse 1, welches hier geöffnet dargestellt ist. Figur 2 zeigt eine schematische Darstellung der Vorrichtung aus Figur 1. Das Gehäuse 1 ist nach oben hin durch eine Auffangwanne 2 abgeschlossen. Auf der Auffangwanne 2 ist eine Prozesswanne 6 angeordnet, die auf Rollen gelagert ist, sodass die Prozesswanne 6 auf der Auffangwanne 2 verschoben werden kann. Wie in Figur 2 gezeigt, ist die Prozesswanne kürzer ausgebildet als die Auffangwanne 2 und weist hier ca. die Hälfte der Länge der Auffangwanne 2 auf. Außerdem ist eine Haube 5 vorgesehen, welche um die Auffangwanne 2 herum angeordnet ist. Die Haube 5 ist dabei so ausgebildet, dass sie auch die Prozesswanne 6 umschließt, wenn die Haube 5 und die Auffangwanne 6 übereinander angeordnet sind. Die Haube 5 ist auf Rollen gelagert, sodass die Haube 5 auf der Auffangwanne 2 verschoben werden kann. Die Prozesswanne 6 und die Haube 5 können zueinander verschoben werden, ohne dass sie sich gegenseitig behindern. Hierfür weist die Haube 5 auf einer Seite eine verschließbare Öffnung 81 auf, durch die die Prozesswanne 6 geschoben werden kann. Zudem kann die Haube 5 ein hier nicht gezeigtes Sichtfenster aufweisen. Die Haube 5 ist ebenfalls kürzer als die Auffangwanne 2 ausgebildet und weist hier ebenfalls die Hälfte der Länge der Auffangwanne 2 auf. Die Prozesswanne 6 ist in einer Behandlungsposition positioniert und die Haube 5 ist in einer Einlegeposition positioniert. Auf der Seite der Einlegeposition ist eine Tür 4 vorgesehen, die im offenen Zustand dargestellt ist. Somit ist die Prozesswanne 6 von außen frei zugänglich und es kann ein Leichnam oder ein Kadaver in einem Beschickungskorb 7 von der Seite der Türe 4 oder alternativ von oben in die Prozesswanne 6 eingelegt werden. Der Beschickungskorb 7 mit dem Leichnam bzw. Kadaver kann mit einem nicht gezeigten Transportwagen an die Türe 4 hingefahren werden, mittels einer höhenverstellbaren Einrichtung des Transportwagens positioniert werden und dann über die geöffnete Türe in die Prozesswanne 6 eingeschoben werden. Der Beschickungskorb 7 wird während des Betriebs an der Prozesswanne 6 verriegelt. Zum Schließen wird die Türe 4 nach oben geklappt. Befindet sich die Haube 5 in der Behandlungsposition über der Prozesswanne 6, schließt diese mit der nach oben geklappten Türe 4 die Prozesswanne 6 nach außen hin ab.

Das Gehäuse 1 ist waagerecht mit Stellfüßen 8 auf dem Boden abgestellt. Zum Boden hin ist zudem eine Leckagewanne 9 zum Auffangen von Leckageflüssigkeit vorgesehen, die durch einen nicht gezeigten Sensor überwacht wird.

Die Vorrichtung wird über eine speicherprogrammierbare Steuerung gesteuert, die auf einem elektronischen Steuergerät 79 abläuft. Das Steuergerät 79 ist in einem Elektroschaltschrank 10 an der Vorrichtung angeordnet. Das elektronische Steuergerät 79 ist mit einem Bediendisplay 3 verbunden, das als Schnittstelle zum Benutzer dient. Das Bediendisplay 3 zeigt dem Benutzer den Stand des Prozesses an und empfängt Eingaben von diesem. Zudem werden dem Benutzer über das Bediendisplay 3 Abfragen angezeigt und dieser kann die Werte bzw. Parameter direkt über das Bediendisplay 3 eingeben oder auswählen. Es sind weitere Signaleinrichtungen vorgesehen, beispielsweise eine Signalampel 18, die aus drei Leuchten besteht und den Status der Vorrichtung durch unterschiedliche Farben und/oder Blinken/Dauerlicht anzeigt, oder eine Hupe 19, die ein akustisches Signal, insbesondere bei einer Störung, erzeugt. In der Vorrichtung sind an beweglichen Komponenten Sicherheitsschalter 77 vorgesehen, die verhindern, dass die Komponenten bewegt, geöffnet oder Ähnliches werden, wenn dadurch Gefahr für den Benutzer entstehen würde, beispielsweise indem Lauge austritt. Ist ein gefahrloser Zugriff durch den Benutzer möglich, geben die Sicherheitsschalter 77 die Komponenten frei. Die Sicherheitsschalter 77 können über das Bediendisplay 3 vom Benutzer gesteuert werden.

Es ist ein Dosierbehälter 11 vorgesehen, in dem beispielsweise Kaliumhydroxid in Form von Granulat als Base vorgehalten wird. Dabei verhindert eine Trennvorrichtung eine vorzeitige Reaktion des Kaliumhydroxids. Der Dosierbehälter 11 weist zudem Sicherheitsschalter und eine Abdeckung auf, die den Benutzer schützen. Der Dosierbehälter 11 ist mit einem Präparationsbehälter 12 verbunden und dosiert die Base entsprechend den Vorgaben in den Präparationsbehälter 12 ein. Das eingebrachte Kaliumhydroxid bildet in Frischwasser, das dem Präparationsbehälter 12 zugeführt wird, Kalilauge, mit der die alkalische Hydrolyse erfolgt. Eine pH-Wert-Messsonde 57, die in einem mit dem Präparationsbehälter 12 verbundenen Leitungssystem angeordnet ist, überwacht den pH-Wert des Prozesswassers. Mehrere Füllstandsensoren 82, 83, 84 im Präparationsbehälter 12 überwachen den Füllstand des Prozesswassers.

Es sind mehrere Düsen 17 in der Haube 5 vorgesehen. Die Düsen 17 und die dazugehörigen Leitungen können in Ausführungsbeispielen auf einem Schlitten angeordnet sein und zur Wartung und Reinigung aus der Haube 5 herausgefahren werden. In Figur 1 sind die Düsen 17 und die dazugehörigen Leitungen zur besseren Übersicht aus der Haube 5 herausgefahren dargestellt. Während des Betriebs befinden sich die Düsen 17 und die dazugehörigen Leitungen an der Decke der Haube 5. Wenn sich die Haube 5, wie in Figur 2 dargestellt, in der Behandlungsposition befindet, sind die Düsen 17 über eine Steckkupplung 71 mit dem Präparationsbehälter 12 verbunden. Wird die Haube 5 verschoben, löst sich die Verbindung über die Steckkupplung 71. Beim Zurückschieben in die Behandlungsposition, greift die Steckkupplung 71 von selbst ein und stellt die Verbindung wieder her. Zudem hält die Stecckupplung 71 die Haube 5 in der Behandlungsposition.

In der Prozesswanne 6 sind weitere Düsen 16 angeordnet, welche den Leichnam bzw. den Kadaver von unten und/oder von der Seite besprühen. Wenn sich die Prozesswanne 6, wie in Figur 2 dargestellt, in der Behandlungsposition befindet sind die Düsen 16 ebenfalls über eine Steckkupplung 70 mit dem Präparationsbehälter 12 verbunden. Wird die Prozesswanne 6 verschoben, löst sich die Verbindung über die Steckkupplung 70. Beim Zurückschieben in die Behandlungsposition, greift die Steckkupplung 70 von selbst ein und stellt die Verbindung wieder her. Zudem hält die Steckkupplung 70 die Prozesswanne 6 in der Behandlungsposition.

Die Prozesswanne 6 weist an einer Seite einen Schieber 80 auf, durch den das Prozesswasser in die Auffangwanne 2 ablaufen kann. Der Schieber 80 ist über das Bedienelement 3 vom Bediener verschließbar, sodass sich das Prozesswasser in der Prozesswanne 6 sammelt, wenn in einem bestimmten Prozessschritt ein Bad erzeugt werden soll. Die weiteren Düsen 16sorgen bei geschlossenem Schieber 80 für eine stetige Zirkulation des Prozesswassers um den Leichnam bzw. den Kadaver.

Der Beschickungskorb 7 weist an den Stellen, an denen sich im eingelegten Zustand die Düsen 16, 17 befinden, eine wasserdurchlässige Struktur, wie z. B. ein Gitter auf, sodass das Prozesswasser ohne Behinderung durch den Beschickungskorb 7 in Kontakt mit der Oberfläche des Leichnams bzw. Kadavers gelangt.

Die Auffangwanne 2 ist in einen Behandlungsabschnitt 20 und in einen Reinigungsabschnitt 21 aufgeteilt. Die Prozesswanne 6 und die Haube 5 befinden sich in ihren Behandlungspositionen über dem Behandlungsabschnitt 20. Im Behandlungsabschnitt 20 und im Reinigungsabschnitt 21 sind Abläufe 22 bzw. 23 angeordnet. Im Ablauf 22 des Behandlungsabschnitts 20 ist ein mehrstufiger Filter 24 angeordnet, der mittels drei Filterstufen Gewebeteile und kleinste Schwebstoffe aus dem Prozesswasser filtert. Die erste Filterstufe ist ein Lochblech, durch das größere Partikel und kleinere Knochen gestoppt werden. Die zweite Filterstufe ist ein engeres Lochblech oder Sieb, durch das kleinere Partikel herausgefiltert werden. Die dritte Filterstufe bildet eine Filtereinheit aus einem Naturmaterial, wie z. B. Baumwolle, die Schwebstoffe und feine Partikel, die zum Leichnam bzw. Kadaver gehören, aufhält. Die Filtereinheit der dritten Filterstufe ist auswechselbar und zur einmaligen Verwendung ausgebildet und kann für jeden Leichnam bzw. Kadaver separat eingesetzt werden. Die Filtereinheit trägt ein Kennzeichen, wie beispielsweise eine Nummer, mit der die Filtereinheit dem Leichnam bzw. Kadaver nach der Hydrolyse zugeordnet werden kann. Das Filtergut sowie die Filtereinheit der dritten Stufe können getrocknet und zusammen mit den Überresten vermahlen werden und schließlich an einen Bestatter, einen Angehörigen oder, im Falle eines Tieres, an dessen Besitzer ausgehändigt werden. Nachdem das Prozesswasser den mehrstufigen Filter 24 durchlaufen hat, sind darin keine Bestandteile des Leichnams bzw. des Körpers mehr enthalten. Im Ablauf 23 befindet sich ein einfacher Filter 25.

Es ist ein Konditionierungsbehälter 13 vorgesehen, in dem das Prozessabwasser nach der Behandlung neutralisiert wird. Hierfür wird Säure in den Konditionierungsbehälter 13 eingeleitet, bis der pH-Wert des Prozessabwassers einen Sollwert unterschreitet. Der Präparationsbehälter 12 ist über eine Leitung mit dem Konditionierungsbehälter 13 verbunden und nach Beendigung des Zersetzungsprozesses wird zumindest ein Teil des Prozessabwassers in den Konditionierungsbehälter 13 geleitet. Eine pH-Wert-Messsonde 51, die in einem mit dem Konditionierungsbehälter 13 verbundenen Leitungssystem angeordnet ist, überwacht den pH-Wert des Prozessabwassers. Mehrere Füllstandsensoren 85, 86 im Konditionierungsbehälter 13 überwachen den Füllstand des Prozessabwassers.

Die Vorrichtung weist zwei Kreiselpumpen 15 und 14 auf, mit denen das Prozesswasser bzw. das Prozessabwasser durch die Leitungssysteme der Vorrichtung gefördert werden. Eine erste Pumpe 14 dient zur Zirkulation des Prozesswassers um den Präparationsbehälter 12, zur Förderung des Prozesswassers vom Präparationsbehälter 12 zu den Düsen 16, 17 und zur Förderung des Prozesswassers vom Präparationsbehälter 12 zum Konditionierungsbehälter 13. Eine zweite Pumpe 15 dient zur Zirkulation des Prozessabwassers um den Konditionierungsbehälter 13 und zum Abpumpen des neutralisierten Prozessabwassers über einen Abwasserabfluss 44 in ein Abwassersystem.

Der Präparationsbehälter 12 und der Konditionierungsbehälter 13 sind unterhalb der Auffangwanne 2 angeordnet, sodass eine Flüssigkeit von dort allein aufgrund der Schwerkraft in die Behälter 12 bzw. 13 fließt.

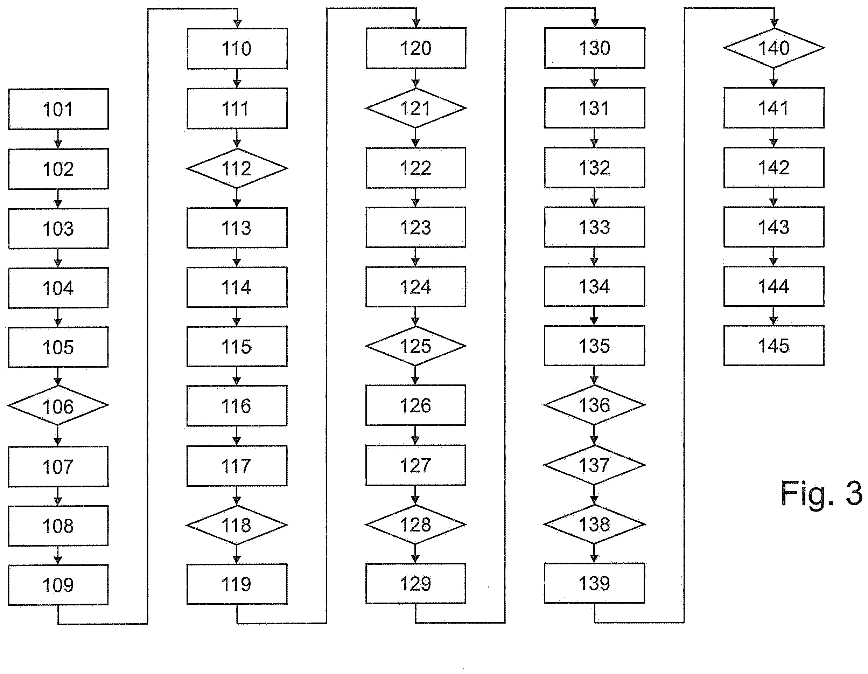

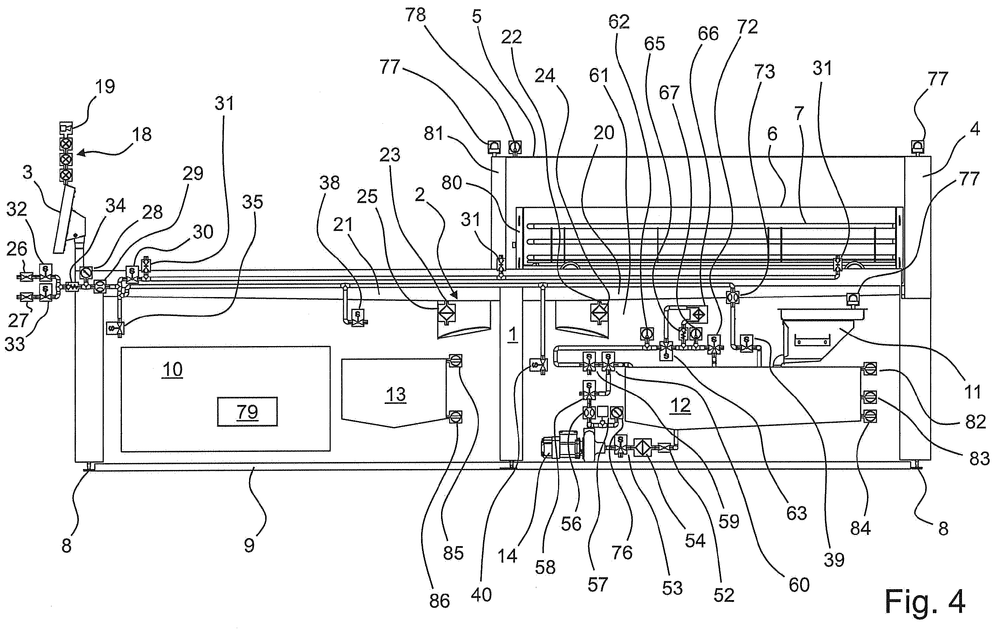

Die Beschreibung der weiteren Komponenten erfolgt anhand der Figuren 4 bis 13, in denen die erfindungsgemäße Vorrichtung in verschiedenen Etappen der alkalischen Hydrolyse gezeigt ist. Zur besseren Übersicht, sind nur die bei dieser Etappe verwendeten Leitungssystemen dargestellt. Nachfolgend wird gleichzeitig das erfindungsgemäße Verfahren, von dem ein Ablaufdiagramm in Figur 3 gezeigt ist, beschrieben.

Für den Fall, dass sich keine Lauge im Präparationsbehälter befindet, wird diese angesetzt 101. Hierfür wird, wie in Figur 4 gezeigt, über einen Frischwasseranschluss dem Präparationsbehälter 12 Frischwasser zugeführt 102, bis eine vorgegeben Wassermenge erreicht ist. In diesem Ausführungsbeispiel sind ein Kaltwasseranschluss 26 mit einem Ventil 32 und ein Warmwasseranschluss 27 mit einem Ventil 33 vorgesehen. Ein Rückschlagventil 34 verhindert den Rückfluss aus dem anschließenden Rohrsystem. Der Wasserdruck des Frischwassers wird durch einen Manometer 28 in der Rohrleitung gemessen. Die Durchflussmenge des Frischwassers wird durch einen elektronischen Wasserzähler 29 in Form eines Durchflussmengenzählers gemessen. Weitere Magnetventile 30, 35, 38, 39, 40 steuern den Zufluss des Frischwassers. Bei Verwendung eines Warmwasseranschlusses 27, wie in diesem Ausführungsbeispiel vorgesehen, werden das Ventil 33 vom Warmwasseranschluss 27 zum Präparationsbehälter 12 und das Ventil 39 geöffnet und der Präparationsbehälter 12 mit warmen Frischwasser befüllt. Nach Erreichen der vorgegebenen Wassermenge, die durch den Durchflussmengenzähler 29 überwacht wird, schließen die Ventile 33 und 39 wieder. Der Füllstandsensor 82 im Präparationsbehälter 12 meldet, wenn dieser voll ist, und verhindert somit ein Überlaufen. Gleichzeitig wird die Base, die in diesem Beispiel Kaliumhydroxid ist, das als Granulat vorliegt, vorbereitet. Alternativ kann auch Natriumhydroxid als Base verwendet werden. Der Benutzer füllt 103 nun eine ausreichende Menge der Base in den Dosierbehälter 11. Hierzu wird der Benutzer über das Bediendisplay 3 aufgefordert. Nach Bestätigung gibt der Sicherheitsschalter 77 den Dosierbehälter 11 frei und der Benutzer kann diesen aus dem Gehäuse 1 herausziehen. Dann schüttet der Benutzer das Kaliumhydroxid mittels einer Schaufel in den Dosierbehälter 11 und schiebt ihn wieder in das Gehäuse 1. Der Dosierbehälter 11 dosiert 104 dann das Kaliumhydroxid, vorzugsweise während des anschließend beschriebenen Zirkulation 105, in den Präparationsbehälter 12. Dort bildet das Kaliumhydroxid in der wässrigen Lösung Kalilauge.

Sowohl für den Fall, dass die Lauge wie vorstehend beschrieben neu angesetzt wurde, als auch für den Fall, dass noch Prozesswasser im Präparationsbehälter 12 vorhanden war, erfolgt eine Zirkulation 105 des Prozesswassers im Präparationsbehälter 12. Am Auslass am unteren Ende des Präparationsbehälters 12 ist ein mechanischer Abstellhahn 52 vorgesehen. Der mechanische Abstellhahn 52 und das 3-Wege-Ventil 53 werden geöffnet und die erste Pumpe 14 saugt mit einer Leistung von 50 % das Prozesswasser durch den Filter 54 und schließlich zurück in den Präparationsbehälter 12. Dabei nehmen ein Durchflussmengenzähler 56, die pH-Wert-Messsonde 57 und ein Manometer 76 in der Leitung die entsprechenden Werte des Prozesswassers auf. In dieser Ausführungsform wird das Prozesswasser zudem erhitzt, sodass es durch einen Durchlauferhitzer 66 gepumpt wird. Hierfür werden die Ventile 58, 59, 60 und 63 entsprechend geschaltet. Das 3-Wege-Ventil 72 leitet das Prozesswasser dann in den Präparationsbehälter 12 zurück. Die aktuelle Temperatur wird dabei durch einen stromaufwärts angeordneten Temperaturfühler 62 und durch einen stromabwärts angeordneten Temperaturfühler 67 gemessen. Die aktuelle Temperatur wird mit einer vorgegebenen Temperatur verglichen. Wird die vorgegebene Temperatur - beispielsweise 98 °C - erreicht, wird das Prozesswasser nicht mehr durch den Durchlauferhitzer 66 gepumpt, indem entweder das Ventil 63 umgeschaltet wird (ein Rückschlagventil 65 verhindert den Rücklauf in den Durchlauferhitzer 66) oder das 3-Wege-Ventil 60 in Richtung des Präparationsbehälter 12 geschaltet wird. Bei dem Fall, dass noch Prozesswasser im Präparationsbehälter 12 vorhanden war, kann die Zirkulation 105 auch zuerst ohne Heizen erfolgen und das Prozesswasser über das 3-Wege-Ventil 60 direkt in den Präparationsbehälter 12 zurückgeleitet werden. Durch die Zirkulation 105 wird sichergestellt, dass die Base bzw. die Lauge sich gleichmäßig im Prozesswasser verteilt. Wenn die pH-Messsonde 57 einen pH-Wert bei einem Sollwert - beispielsweise 12 - misst 106, ist die Lauge vorbereitet und das Prozesswasser einsatzbereit. Dies über das Bediendisplay 3 angezeigt.

Wenn die pH-Wert-Messsonden 57 im Betrieb einen pH-Wert des Prozesswassers messen, der unter einem Sollwert für den pH-Wert - beispielsweise 12 - liegt, wird der aktuelle Förderung unterbrochen und eine Meldung über das Bediendisplay 3 ausgegeben. Der Dosierbehälter 11 dosiert eine weitere Menge Base in den Präparationsbehälter 12 ein und das Prozesswasser wird erneut zirkuliert 105. Wird der pH-Sollwert wieder erreicht, wird das Verfahren mit an dem vorherigen Stand weitergeführt.

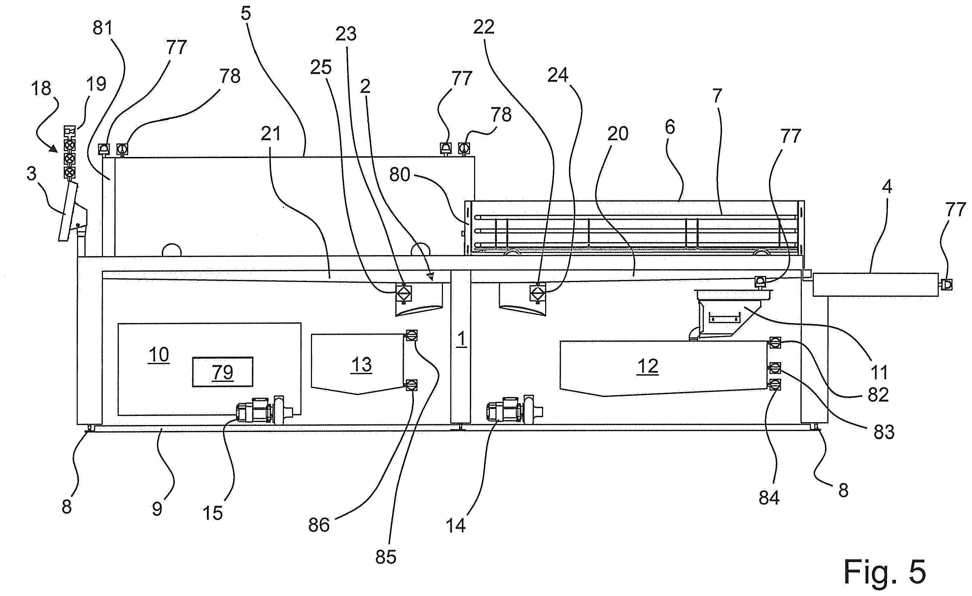

Für den Einlegeprozess des Leichnams bzw. des Kadavers, wie in Figur 5 gezeigt, wird die Haube 5 in die Einlegeposition über den Reinigungsabschnitt 21 der Auffangwanne 2 geschoben 107 und die Türe 4 geöffnet 108. Hierfür werden nach Bestätigung einer entsprechenden Aufforderung am Bediendisplay 3 die Sicherheitsschalter 77 gelöst. In die nun freigelegte Prozesswanne 6 wird der Leichnam bzw. der Kadaver eingelegt 109. Hierbei kann ein Beschickungskorb 7 (oder bei Tierkadavern mehrere Beschickungskörbe) mit dem Kadaver oder Leichnam bestückt werden und der Beschickungskorb 7 wird in die Prozesswanne 6 eingelegt 109 und an dieser verriegelt. Dabei darf nur der reine Körper in die Vorrichtung eingebracht werden. Ein Sarg, Kleidung, Dreingaben, Verpackung etc. werden zuvor entfernt. Beim Einlegen 109 können Hilfsmittel, wie z. B. der Transportwagen, verwendet werden.

Anschließend wird die Türe 4 geschlossen 110 und die Haube 5 wieder in die Behandlungsposition geschoben 111. Dabei stellen die Schnellkupplungen 70 und 71 einerseits die Verbindung der Rohrleitungen zwischen dem Präparationsbehälter 12 und den Düsen 16 und 17 her und andererseits wird eine Verriegelung der Haube 5 und der Türe 4 bewirkt. Die Sicherheitsschalter 77 an der Haube 5 und an der Tür 4 werden aktivieret und verhindern ein unbeabsichtigtes Öffnen, was dem Schutz des Benutzers dient. Sollte während des Betriebs ein Öffnen nötig werden, kann dies über das Bediendisplay 3 veranlasst werden. Dann wird das laufende Verfahren unterbrochen und die Düsen 16, 17 werden, wie später beschrieben, mit Frischwasser gespült und nach einer vorgegebenen Zeit für das Abtropfen, die Türe 4 und die Haube 5 freigegeben.

Generell können das Ansetzen des Prozesswassers gemäß den Schritten 101 bis 106 und der Einlegeprozess des Leichnams bzw. Kadavers gemäß den Schritten 107 bis 111 auch simultan erfolgen oder in umgekehrter Reihenfolge stattfinden.

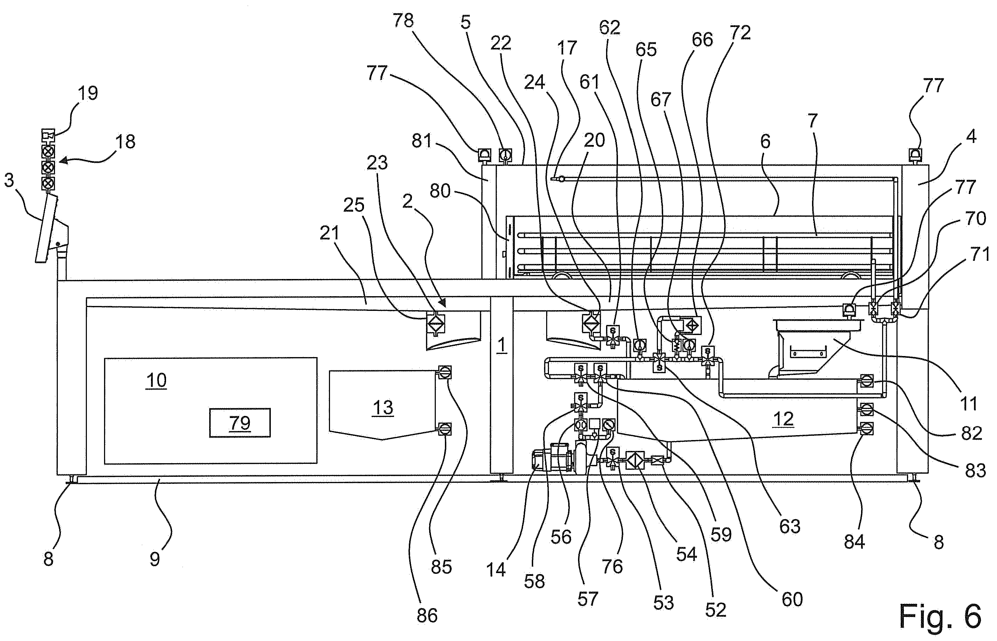

Figur 6 zeigt die Etappe, bei der die alkalische Hydrolyse ausgeführt wird. Nach Bestätigung einer Abfrage 112, ob die Türe 4 und die Haube 5 geschlossen sind, kann am Bediendisplay 3 die Aquamation gestartet werden. Die Zirkulation 105 des Prozesswassers wird beendet und das Prozesswasser zu den Düsen 16 und 17 gefördert 113. Hierfür werden der Abstellhahn 52 geöffnet und die 3-Wege-Ventile 58, 59, 60 und 63 in Richtung Durchlauferhitzer 66 geschalten, Die erste Pumpe 14 wird mit voller Leistung betrieben und das Prozesswasser durch den Durchlauferhitzer 66 geführt. Auch hier nehmen der Durchflussmengenzähler 56, die pH-Wert-Messsonde 57 und der Manometer 76 in der Leitung die entsprechenden Werte des Prozesswassers auf. Die Temperaturfühler 62 und 67 messen wiederum die aktuelle Temperatur. Kann die vorgegebene Temperatur - beispielsweise 98 °C - nicht erreicht werden, wird erneut die Zirkulation 105 über den Durchlauferhitzer 66 in den Präparationsbehälter 12 durchgeführt. Wenn die vorgegebene Temperatur erreicht wurde, wird das Ventil 63 so umgeschaltet, dass das Prozesswasser nicht zum Durchlauerhitzer 66 strömt, wobei das Rückschlagventil 65 ein Zurückfließen in den Durchlauferhitzer 66 verhindert. Zudem wird das 3-Wege-Ventil 72 so geschaltet, dass das Prozesswasser kontinuierlich zu den angeschlossenen Schnellkupplungen 70, 71 geführt wird. Das Prozesswasser wird einerseits über die Schnellkupplung 71 zu den Düsen 17 in der Haube 5 und andererseits über die Schnellkupplung 70 zu den Düsen 16 in der Prozesswanne 6 geführt und dort auf den Leichnam bzw. den Kadaver in der Prozesswanne 6 gesprüht 114. Es beginnt der Zersetzungsprozess, bei dem die Kalilauge den Leichnam bzw. Kadaver zersetzt.

Der Schieber 80 wird - wenn dies nicht bereits der Fall ist - geöffnet 115, sodass das Prozesswasser in die Auffangwanne 2 abfließen kann. Das Prozesswasser, mit dem der Leichnam bzw. der Kadaver besprüht wird, sammelt sich im Behandlungsabschnitt 20 der Auffangwanne 2 und läuft aufgrund der schrägen Ebene der Auffangwanne 2 zum Ablauf 22. Von dort wird das Prozesswasser in den Präparationsbehälter 12 abgeführt. Aufgrund der Schwerkraft läuft das Prozesswasser durch den Ablauf 22 im Behandlungsabschnitt 20, durch den mehrstufigen Filter 24 und über das geöffnete Ventil 61 in den Präparationsbehälter 12. Der mehrstufige Filter 24 filtert dabei die restlichen Bestandteile des Leichnams bzw. des Kadavers aus dem Prozesswasser. Das Prozesswasser wird dann wie oben beschrieben kontinuierlich erneut zu den Düsen 16, 17 gefördert 113. In der Haube 5 überwacht ein Temperaturfühler 78 kontinuierlich die Temperatur. Die Daten können als Nachweis für eine konstante Temperatur gegenüber externen Stellen dienen. Zudem wird der Füllstand im Präparationsbehälter 12 kontinuierlich durch die Füllstandsensoren 82, 83, 84 überwacht. Sinkt der Füllstand unter einen vom Füllstandsensor 83 überwachten Schwellenwert, wird eine Meldung über das Bediendisplay 3 angezeigt und die Leistung der ersten Pumpe 14 gedrosselt, sodass deren Durchfluss reduziert wird und weniger Prozesswasser entnommen wird. Nach einer festlegbaren Zeit, die vom Fortschritt des Zersetzungsprozesses abhängt, wird der Schieber 80 geschlossen 116, sodass sich das Prozesswasser in der Prozesswanne 6 sammelt und ein Bad erzeugt. Dabei läuft weiterhin Prozesswasser über die Auffangwanne 2 zum Präparationsbehälter 13 zurück. Die Düsen 16 in der Prozesswanne 6 sorgen jetzt für eine stetige Zirkulation des Prozesswassers im Bad.

Nun wird die berechnete Prozesszeit abgewartet 117. Die Prozesszeit ist abhängig vom Gewicht des Leichnams bzw. Kadavers, der Temperatur und dem Druck sowie der Agitation und beträgt normalerweise zwischen 3 und 20 Stunden. Optional kann die verbleibende Prozesszeit auf dem Bediendisplay 3 angezeigt werden. Über das nicht gezeigte Sichtfenster in der Haube 5 kann der Zersetzungsprozess durch den Benutzer überwacht werden. Sollte die berechnete Prozesszeit nicht ausreichen, kann der Benutzer diese über das Bediendisplay 3 verlängern. Wenn der Benutzer die Abfrage 118, ob die Aquamation beendet ist, bestätigt, wird der Schieber 80 geöffnet 119, sodass das Prozessabwasser vollständig in die Auffangwanne 2 abläuft. Außerdem wird das Ventil 60 so geschaltet, dass das Prozessabwasser in den Präparationsbehälter 12 zirkuliert 120.

Während des Betriebs werden die Prozessdaten, wie z. B. gemessene Temperaturen, gemessene pH-Werte, gemessene Drücke und/oder die Prozesszeit im Steuergerät 79 gespeichert und können ebenfalls über das Bediendisplay 3 angezeigt werden. Unterschreitet der pH-Wert einen Schwellenwert, beispielsweise 10,5, kann eine Alarmmeldung, insbesondere als akustisches Signal z. B. mittels der Hupe 19 und/oder als visuelles Signal z. B. über die Signalampel 18 ausgegeben werden, damit der Benutzer entsprechend einschreiten kann und der Fehler behoben werden kann.

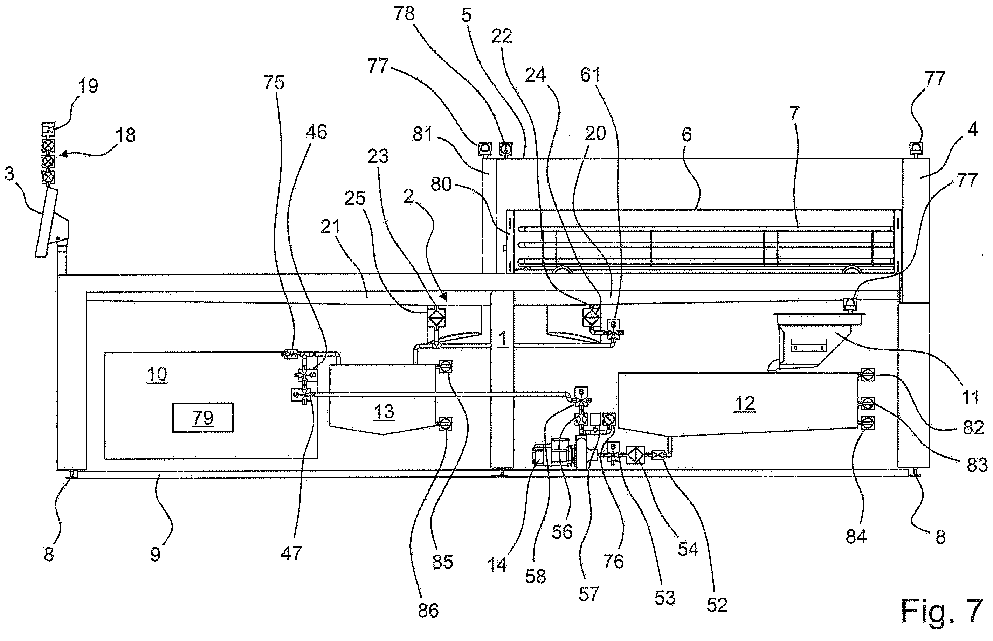

Im Folgenden wird die Nachbehandlung und die Reinigung der Vorrichtung beschrieben. Bereits im Betrieb oder nachdem die Aquamation beendet ist, erfolgt eine Abfrage 121, ob ein Umpumpen des Prozessabwassers aus dem Präparationsbehälter 12 in den Konditionierungsbehälter 13 stattfinden soll. Wird dies bestätigt, so wird ein Teil des Prozessabwassers - ca. ein Drittel des Prozessabwassers im Präparationsbehälter 12 - in den Konditionierungsbehälter 13 durch die erste Pumpe 14 umgepumpt 122, wie in Figur 7 dargestellt. Das 3-Wege-Ventil 58 wird so geschaltet, dass das Prozessabwasser aus dem Präparationsbehälter 12 über den geöffneten Abstellhahn 52 entnommen wird und über die entsprechend geschalteten Ventile 47 und 46 in den Konditionierungsbehälter 13 gefördert wird. Dabei misst der Durchflussmengenmesser 56 die Menge des Prozessabwassers und nach Erreichen der gewünschten Menge wird die Verbindung zwischen dem Präparationsbehälter 12 und dem Konditionierungsbehälter durch Schließen der Ventile 58 und 47 getrennt. Das 3-Wege-Ventil 58 wird so eingestellt, dass die Zirkulation 120 fortgesetzt wird. Die im Präparationsbehälter 12 verbleibende Menge Prozesswasser kann für eine neue alkalische Hydrolyse eines weiteren Leichnams bzw. Kadavers verwendet werden. Für das erneute Ansetzen 101 der Lauge, wird in diesem Fall eine Menge an Frischwasser zugeführt, die der entnommenen Menge Prozessabwasser entspricht. Die weiteren Schritte 102 bis 106 werden gleich ausgeführt.

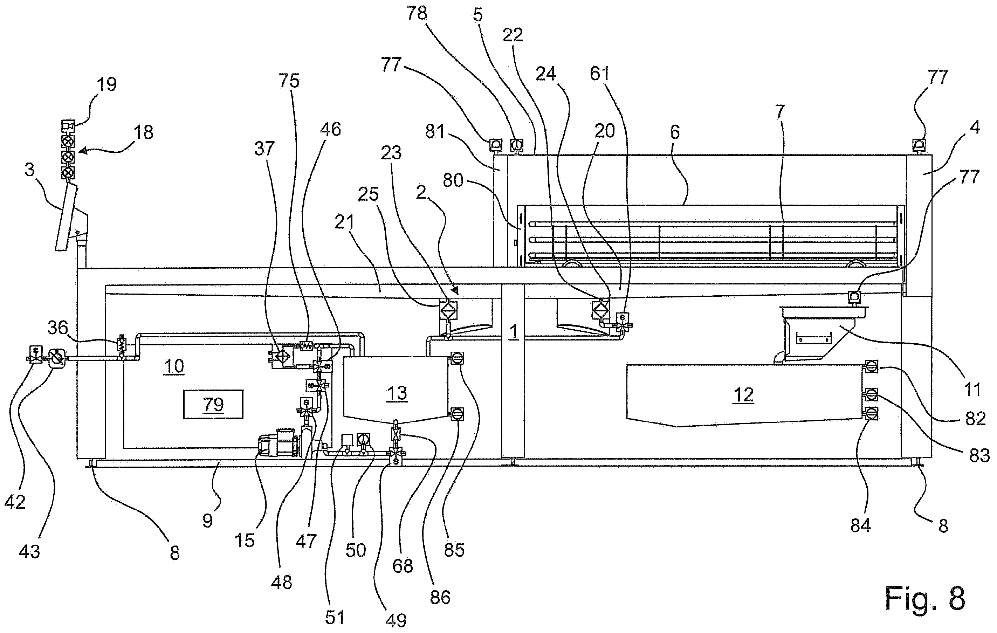

In Figur 8 ist das Prozessabwasser aus dem Präparationsbehälter 12 im Konditionierungsbehälter 13 gelagert. Es erfolgt die Konditionierung 123 des Prozessabwassers. Hierfür wird eine mit einem Säurezulauf 42 verbundene Säuredosierpumpe 43 eingeschaltet und diese führt dem Konditionierungsbehälter 13 Säure, insbesondere eine 50%-ige Zitronensäure, zu. Die Säuredosierpumpe 43 ist auf einen Durchfluss von 110 Liter pro Stunde ausgelegt. Die Säure neutralisiert die Lauge im Prozessabwasser, sodass der pH-Wert sinkt. Der pH-Wert wird dabei kontinuierlich mittels der Messsonde 51 gemessen. Die Säure wird solange zugeführt, bis der pH-Wert des Prozessabwassers einen Schwellenwert für den pH-Wert unterschritten hat. Der pH-Wert unterliegt also einer Regelung, bei der die Säuredosierpumpe 43 als Regelstrecke und die Messsonde 51 als Messtrecke dient.

Zudem findet eine Zirkulation 124 des Prozessabwasser kontinuierlich durch den Konditionierungsbehälter 13 und das zugehörige Rohrsystem statt, wie ebenfalls in Figur 8 dargestellt. Am Auslass am unteren Ende des Konditionierungsbehälters 13 ein mechanischer Abstellhahn 68 vorgesehen. Der mechanische Abstellhahn 68 und das 3-Wege-Ventil 49 werden geöffnet. Die zweite Pumpe 15 wird gestartet und läuft mit reduzierter Leistung, beispielsweise bei 50%. Die zweite Pumpe 15 pumpt das Prozessabwasser aus dem Auslass am unteren Ende des Konditionierungsbehälters 13 ab, fördert es über die entsprechend geschalteten Ventile 48, 47, 46 zu dem Kühler 37 und von dort aus am oberen Ende des Konditionierungsbehälters 13 wieder in diesen zurück. Dadurch wird das Prozessabwasser durchmischt und mit der Säure in Kontakt gebracht. Der Kühler 37 kühlt die Temperatur des Prozessabwassers ab. Die Abwärme kann beispielsweise für eine Erwärmung des Prozesswassers im Präparationsbehälter 12 verwendet werden. Die aktuelle Temperatur wird dabei durch einen Temperaturfühler 50 überwacht. Die aktuelle Temperatur wird mit einem Temperatur-Schwellenwert verglichen. Wird der Temperatur-Schwellenwert - beispielsweise 40 °C - unterschritten, wird das Prozesswasser nicht mehr durch den Kühler 37 gepumpt, indem das Ventil 46 umgeschaltet wird (ein Rückschlagventil 75 verhindert den Rücklauf in den Kühler 37).

Ergibt ein Vergleich 125, dass der gemessene pH-Wert den Sollwert für den pH-Wert - beispielsweise 9,5 - unterschritten hat, wird die Säuredosierpumpe 43 abgeschaltet 126. Im Anschluss findet eine Spülung 127 des Rohrsystems für den Säurezulauf statt, bei der die Rohrleitungen mit Frischwasser gereinigt werden. Hierfür werden das Ventil 32 vom Kaltwasseranschluss 26 und das Ventil 35 geöffnet, sodass Kaltwasser in das Rohrsystem eingeleitet werden kann. Ein Rückschlagventil 36 verhindert einen Rückfluss in das Frischwassersystem. Das Spülwasser wird in den Konditionierungsbehälter 13 abgeleitet und trägt dort zusätzlich zur Kühlung des Prozessabwassers bei.

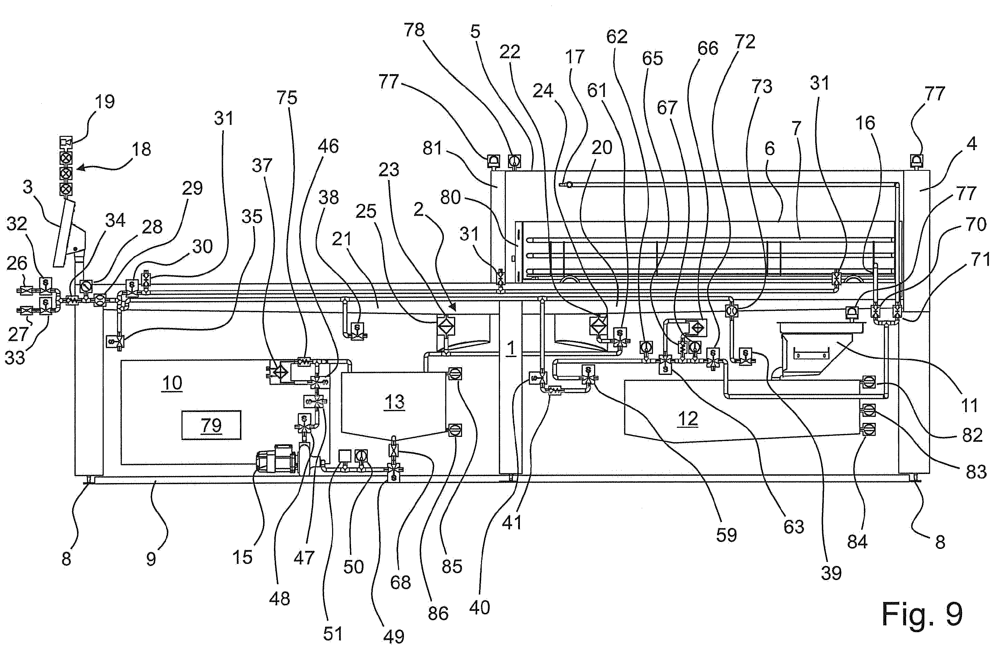

In einer weiteren Abfrage 128 wird geprüft, ob die Düsen 16, 17 gespült werden sollen. Nach Bestätigung durch den Benutzer werden die Düsen 16, 17 und das zugehörige Rohrsystem gespült 129, wie in Figur 9 dargestellt. Hierfür werden das Ventil 32 vom Kaltwasseranschluss 26 und das Ventil 40 geöffnet, sodass Kaltwasser in das Rohrsystem eingeleitet werden kann. Zudem werden die Ventile 59, 63 und 72 so geöffnet, dass das Kaltwasser bis in die Düsen 16, 17 geleitet wird. Um den Durchflusserhitzer 66 zu spülen, kann das Ventil 63 umgeschaltet werden. Der Schieber 80 an der Prozesswanne 6 bleibt während der Reinigung geöffnet. Das durch die Düsen 16, 17 ausgeschiedene Spülwasser sammelt sich dann im Behandlungsabschnitt 20 der Auffangwanne 2 und wird, indem das 3-Wege-Ventil 61 in Richtung Konditionierbehälter 13 öffnet, aufgrund der Schwerkraft über den Ablauf 22 zum Konditionierbehälter 13 abgeführt. Dort wird es der Zirkulation 124 zugeführt. Nach einer vorgegebenen Menge Frischwasser, die durch den Durchflussmengenzähler 29 gemessen wird, wird das Spülen beendet und das Ventil 40 schließt. Dieser Schritt 129 kann auch ausgeführt werden, wenn die Haube 5 oder die Türe 4 während der Aquamation geöffnet werden soll.

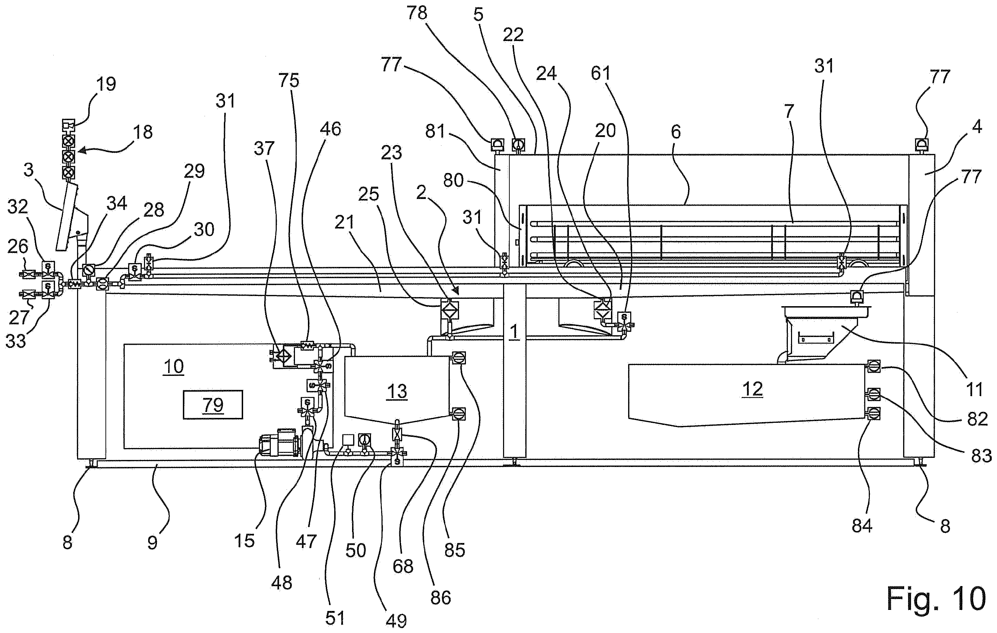

Nach dem Spülvorgang, kann ein Bediener die Überreste aus der Prozesswanne 6 entnehmen. Hierfür geben die Sicherheitsschalter 77 die Haube 5 und die Prozesswanne 6 frei. Der Bediener öffnet die Öffnung 81 der Haube 5 und schiebt 130 die Prozesswanne 6, wie in Figur 10 gezeigt, in die Reinigungsposition, sodass sie sich über dem Reinigungsabschnitt 21 der Auffangwanne 2 befindet. Das Frischwassersystem weist Steckkupplungen 31 auf, an die eine Handbrause angeschlossen werden kann. Die Ventile 30 an den Steckkupplungen 31 können durch Tastendruck geöffnet und geschlossen werden. Für eine manuelle Reinigung 131 der Überreste schließt der Benutzer eine Handbrause an eine der Steckkupplungen 31 an und öffnet das entsprechende Ventil 30. Das Ventil 32 ist weiterhin geöffnet, sodass Kaltwasser zu der Steckkupplung 31 geführt wird. Dann führt der Benutzer die manuelle Reinigung 131 der Überreste im Beschickungskorb 7 mit Frischwasser durch, während sich die Prozesswanne 6 in der Reinigungsposition befindet. Das Reinigungswasser sammelt sich im Reinigungsabschnitt 21 der Auffangwanne 2, läuft aufgrund der schrägen Ebene der Auffangwanne 2 zum Ablauf 25 und fließt aufgrund der Schwerkraft durch den Ablauf 25 und durch den Filter 26 in den Konditionierungsbehälter 13. Dort wird es ebenfalls der Zirkulation 124 zugeführt. Nach der manuellen Reinigung 131 entnimmt 132 der Benutzer die Überreste aus dem Beschickungskorb 7. Die Entnahme 132 der Überreste an der Reinigungsposition erfolgt somit an einer anderen Position als der Leichnam bzw. der Kadaver in der Behandlungsposition eingelegt wurde. Die beiden Positionen können durch eine Sichtschutzwand außerhalb der Vorrichtung voneinander getrennt sein, sodass Angehörige, die sich auf der Seite der Behandlungsposition befinden, die Reinigung 131 und die Entnahme 132 der Überreste sowie weitere Reinigungsschritte 133 nicht wahrnehmen. Die Knochen werden, wie unten beschrieben, extern zermahlen 135.

Anschließend führt der Benutzer mit Hilfe der Handbrause eine manuelle Reinigung 133 der Prozesswanne 6, der Haube 5 und der Auffangwanne 2 mit Frischwasser durch. Dabei kann die Handbrause auch auf die anderen Stecckupplungen 31 umgesteckt werden. Für die Reinigung der Auffangwanne 2 können die Haube 5 und die Prozesswanne 6 verschoben werden (nicht separat dargestellt). Das Reinigungswasser, welches bei der manuellen Reinigung 133 der Prozesswanne 6, der Haube 5 und der Auffangwanne 2 anfällt, wird dann aufgrund der Schwerkraft durch den Ablauf 23 im Reinigungsabschnitt 21 und/oder durch den Ablauf 22 im Behandlungsabschnitt 20 in den Konditionierungsbehälter 13 abgeführt. Dort wird es ebenfalls der Zirkulation 124 zugeführt. Nach dem Reinigen der Auffangwanne 2 wird der mehrstufige Filter 24 entnommen. Die auswechselbare Filtereinheit der dritten Filterstufe des Filters 24 wird mitsamt den darin befindlichen Überresten über das Kennzeichen an der Filtereinheit dem Leichnam bzw. Kadaver zugeordnet 134. Die Filtereinheit und die Überreste werden getrocknet und zusammen mit den entnommenen Knochen extern zermahlen 135 und dann an einen Bestatter, einen Angehörigen oder, im Falle eines Tieres, an dessen Besitzer ausgehändigt.

Im Konditionierungsbehälter 13 sind nun das Prozessabwasser, das Spülwasser und das Reinigungswasser gesammelt. Zur Vereinfachung wird diese Mischung in der nachfolgenden Beschreibung als Prozessabwasser bezeichnet. Meldet der Füllstandsensor 85, dass zu viel Prozessabwasser im Konditionierungsbehälter 13 vorhanden ist, wird kein Prozesswasser oder Abwasser mehr in den Konditionierungsbehälter 13 gepumpt. Das gesamte Prozessabwasser wird wie oben beschrieben konditioniert 123 und zirkuliert 124. Die pH-Wert-Messsonde 51 im Leitungssystem des Konditionierungsbehälters 13 überwacht kontinuierlich den pH-Wert und der Temperaturfühler 50 überwacht kontinuierlich die Temperatur des Prozessabwassers. Ergibt ein Vergleich 136, dass der gemessene pH-Wert des Prozessabwassers unter einem Schwellenwert für den pH-Wert, der in diesem Beispiel 9,5 beträgt, liegt, und ergibt ein Vergleich 137, dass die Temperatur des Prozessabwassers einen Temperaturschwellenwert, der hier bei 40 °C liegt, unterschritten hat, kann das konditionierte Prozessabwasser ausgelassen werden. In einer Abfrage 138 wird geprüft, ob das Abwassersystem bereit ist.

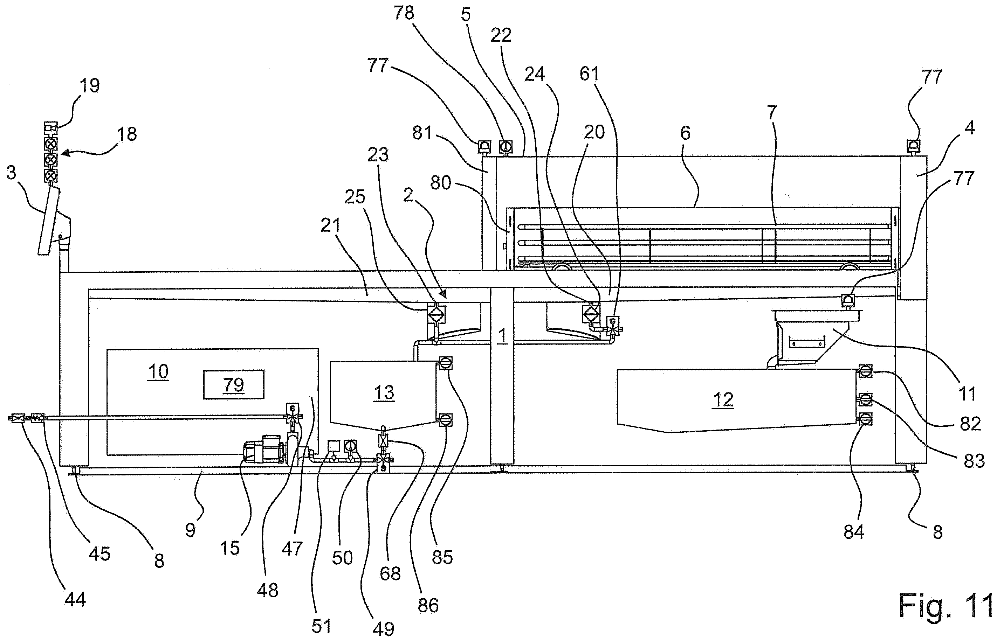

Nach der Bestätigung wird das konditionierte Prozessabwasser, wie in Figur 11 dargestellt, aus dem Konditionierungsbehälter 13 mittels der zweiten Pumpe 15 über den Abwasserabfluss 44 abgepumpt 139. Hierfür wird die zweite Pumpe 15 mit reduzierter Leistung - beispielsweise bei 60% - betrieben und das 3-Wege-Ventil 48 so geschaltet, dass das konditionierte Prozessabwasser aus dem Auslass am unteren Ende des Konditionierungsbehälters 13 über den geöffneten Abstellhahn 68 und das geöffneten Ventil 49 zum Abwasserabfluss 44 geführt wird. Ein Rückschlagventil 45 verhindert den Rückfluss. Meldet der Füllstandsensor 86 im Konditionierungsbehälter 13, dass dieser leer gelaufen ist, wird die zweite Pumpe 15 gestoppt und das Ventil 48 zurückgeschaltet. Das Abpumpen 139 in den Abwasserabfluss 44 wird schließlich noch am Bediendisplay 3 bestätigt.

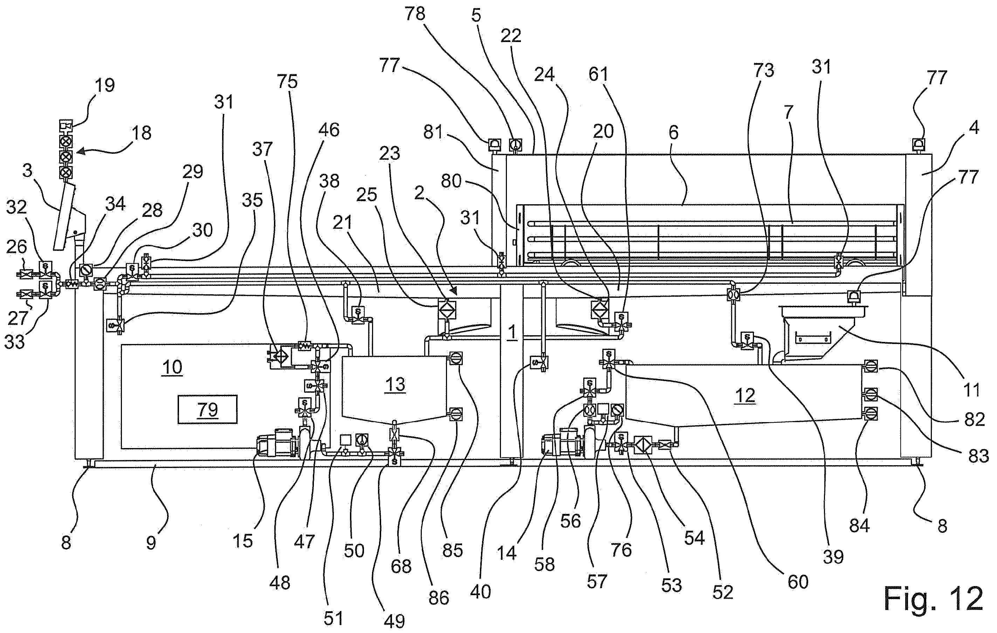

Es kann vorgesehen sein, die Vorrichtung komplett zu entleeren, wie nachfolgend beschrieben und in Figur 12 gezeigt. Es erfolgt eine Abfrage 140, ob ein Spülen der Behälter 12, 13 bereit ist. Die Behälter 12, 13 können alternativ auch einzeln gereinigt werden. Bei Bestätigung werden der Dosierbehälter 11 und der Präparationsbehälter 12 entleert 141. Der Dosierbehälter 11 kann manuell entleert werden, sodass verbliebenes Kaliumhydroxid erneut verwendet werden kann. Das Prozesswasser, welches sich im Präparationsbehälter 12 befindet, wird, wie in Schritt 122 beschrieben, vollständig in den Konditionierungsbehälter 13 umgepumpt. Anschließend findet ein Spülen 142 des Präparationsbehälters 12 mit Frischwasser statt. Hierfür öffnen die Ventile 32 und 39, sodass Kaltwasser in den Präparationsbehälter 12 strömt. Ist eine vorgegebene Menge Frischwasser in den Präparationsbehälter 12 geströmt, schließt das Ventil 39. Das Reinigungswasser wird durch das Leitungssystem, welches bei der Zirkulation 120 des Prozesswassers verwendet wurde, geleitet, indem die Ventile 53, 58 und 60 entsprechend geschaltet werden und die erste Pumpe 14 verwendet wird. Schließlich wird das Ventil 58 umgeschaltet und das Reinigungswasser wird wie im Schritt 122 beschrieben, in den Konditionierungsbehälter 13 umgepumpt 143. Wenn der Füllstandsensor 84 anzeigt, dass der Präparationsbehälter 12 leer gelaufen ist, wird die erste Pumpe 14 gestoppt. Anschließend findet ein Spülen 144 des Konditionierungsbehälters 13 mit Frischwasser statt. Zusätzlich zum Ventil 32 öffnet das Ventil 38 und Kaltwasser strömt in den Konditionierungsbehälter 13. Ist eine vorgegebene Menge Frischwasser in den Konditionierungsbehälter 12 geströmt, schließen die Ventile 32 und 38. Das Reinigungswasser wird durch das Leitungssystem, welches bei der Zirkulation 124 des Prozessabwassers verwendet wurde, geleitet, indem der Abstellhahn 68 geöffnet wird, die Ventile 49, 48, 47 und 46 entsprechend geschaltet werden und die zweite Pumpe 15 verwendet wird. Um den Kühler 37 zu spülen, kann das Ventil 46 umgeschaltet werden. Schließlich wird das Ventil 48 umgeschaltet und das Reinigungswasser wird wie im Schritt 139 beschrieben, über den Abwasserabfluss 44 abgepumpt 145. Wenn der Füllstandsensor 86 anzeigt, dass der Konditionierungsbehälter 13 leer gelaufen ist, wird die zweite Pumpe 15 gestoppt.

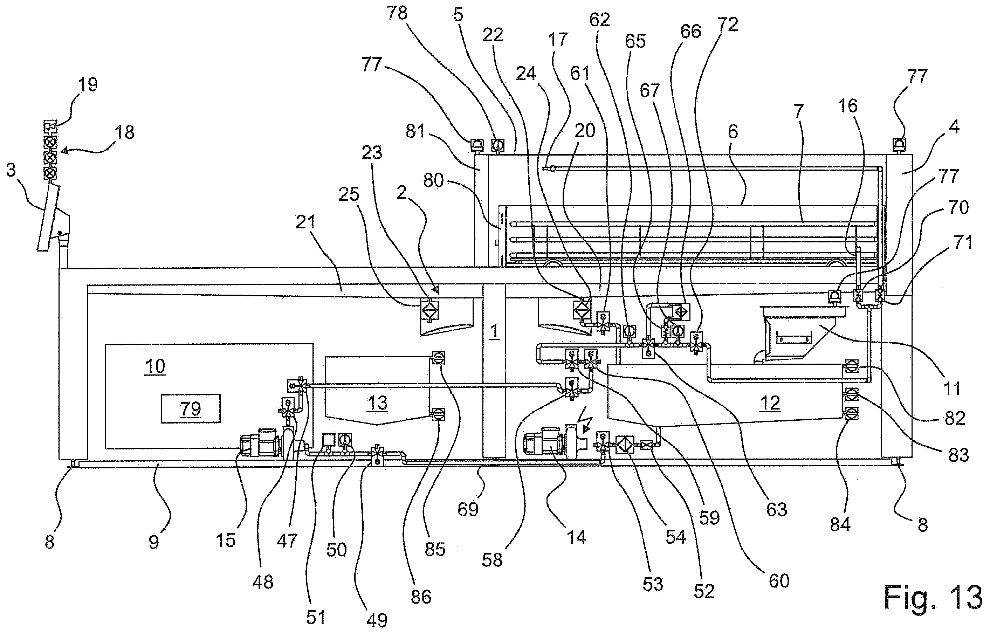

In Figur 13 ist ein Notbetrieb für die Etappe der Aquamation dargestellt, bei der eine Pumpe, in diesem Fall die erste Pumpe 14 ausgefallen ist. Die Ventile 53, 49 und 48 werden so geschaltet, dass das Prozesswasser bei geöffnetem Abstellhahn 52 aus dem Auslass am unteren Ende des Präparationsbehälters 12 über die Überbrückungsleitung 69 zur zweiten Pumpe 15 und von dort über die entsprechend geschalteten Ventile 48, 47 und 58 zurück in das Leitungssystem zu den Düsen 16, 17 geführt wird. Die Ventile 60, 59, 63 und 72 werden wie in Figur 6 beschrieben geschaltet.

## Patentansprüche

1. Vorrichtung zur alkalischen Hydrolyse von Leichnamen und/oder Kadavern, aufweisend:
- eine Prozesswanne (6), in die der Leichnam oder der Kadaver einbringbar ist;
- eine Haube (5), welche die Prozesswanne (6) umschließt;
- einen Präparationsbehälter (12);
- zumindest einen Frischwasserzufluss (26, 27);
- zumindest eine Düse (16, 17) in der Haube (5) und/oder in der Prozesswanne (6), die mit dem Präparationsbehälter (12) verbindbar ist;
- einen Abwasserabfluss (44);
- zumindest eine Pumpe (15, 14); und
- eine speicherprogrammierbare Steuerung (79), welche die Vorrichtung steuert
**dadurch gekennzeichnet, dass** eine Auffangwanne (2) unterhalb der Prozesswanne angeordnet ist und dass die Prozesswanne (6) zu der Auffangwanne (2) beweglich zwischen einer Behandlungsposition, in der die Prozesswanne (6) über einem Behandlungsabschnitt (20) der Auffangwanne (2) angeordnet ist, der mit dem Präparationsbehälter (12) verbunden ist, und einer Reinigungsposition, in der die Prozesswanne (6) über einem Reinigungsabschnitt (21) der Auffangwanne (2) angeordnet ist, der mit dem Abwasserabfluss (44) verbunden ist, gelagert ist

2. Vorrichtung nach Anspruch 1, **gekennzeichnet durch** einen Konditionierungsbehälter (13), der über zumindest eine Leitung mit dem Präparationsbehälter (12), durch die ein Prozessabwasser zum Konditionierungsbehälter (13) geführt wird, wobei der Konditionierungsbehälter (13) eine Einrichtung zur Neutralisierung der Lauge im Prozessabwasser aufweist.

3. Vorrichtung nach Anspruch 2, **dadurch gekennzeichnet, dass** die Einrichtung zur Neutralisation der Lauge eine Dosiereinrichtung (42) zum Einleiten von flüssiger oder fester Säure in den Konditionierungsbehälter (13) ist.

4. Vorrichtung nach einem der Ansprüche 2 oder 3, **dadurch gekennzeichnet, dass** eine erste Pumpe (14) zur Förderung über den Präparationsbehälter (12) und/oder eine zweite Pumpe (15) zur Förderung über den Konditionierungsbehälter (13) vorgesehen ist.

5. Vorrichtung nach einem der vorhergehenden Ansprüche, **dadurch gekennzeichnet, dass** die Haube (5) zu der Prozesswanne (6) beweglich zwischen einer Einlegeposition, in der die Prozesswanne (6) von außen frei zugänglich ist, und einer Behandlungsposition, in der die Haube (5) die Prozesswanne (6) umschließt, gelagert ist.

6. Vorrichtung nach einem der vorhergehenden Ansprüche, **dadurch gekennzeichnet, dass** ein mehrstufiger Filter (24) in einem Ablauf für das Prozesswasser angeordnet ist, der eine auswechselbare Filtereinheit aufweist.

7. Vorrichtung nach Anspruch 6, **dadurch gekennzeichnet, dass** die auswechselbare Filtereinheit zur einmaligen Verwendung ausgebildet ist und ein Kennzeichen zur Zuordnung des Leichnams bzw. Kadavers aufweist.

8. Vorrichtung nach einem der vorhergehenden Ansprüche, **gekennzeichnet durch** zumindest einen Beschickungskorb (7), der eingerichtet ist, den Leichnam und/oder den Kadaver aufzunehmen und in die Prozesswanne (6) eingelegt zu werden.

9. Vorrichtung nach einem der vorhergehenden Ansprüche, **gekennzeichnet durch** eine Heizung (66), welche mit dem Präparationsbehälter (12) verbunden ist.

10. Vorrichtung nach einem der vorhergehenden Ansprüche, **dadurch gekennzeichnet, dass** zumindest eine Messeinrichtung für eine oder mehrere der folgenden Parameter vorgesehen ist:
- Durchflussmenge des Prozesswassers;
- Temperatur des Prozesswassers;
- pH-Wert des Prozesswassers;
- Menge der zugeführten Base oder Lauge;
- zugeführte Wassermenge;
- Temperatur des Prozessabwassers;
- pH-Wert des Prozessabwassers;
- Leitfähigkeit des Prozessabwassers.

11. Verfahren zur alkalischen Hydrolyse von Leichnamen und/oder Kadavern bestehend aus den folgenden Schritten:
I) Zuführen (102) einer berechneten Wassermenge durch den zumindest einen Frischwasserzulauf (42) in einen Präparationsbehälter (12);
II) Einbringen (105) von Lauge und/oder Base in den Präparationsbehälter (12);
III) Einlegen (109) des Leichnams oder des Kadavers in eine Prozesswanne (6);
IV) Bringen (111) einer Haube (5) in eine Behandlungsposition über die Prozesswanne (6);
V) Besprühen (114) des Leichnams oder des Kadavers durch zumindest eine Düse (16, 17) in der Haube und/oder in der Prozesswanne (6) mit Prozesswasser aus dem Präparationsbehälter (12);
VI) Abführen des Prozesswassers;
VII) Nach der vorgegebenen Prozesszeit, Bewegen (130) der Prozesswanne (6) in eine Reinigungsposition;
VIII) Reinigen (134) der Prozesswanne (6), der Haube (5) und einer Auffangwanne (2);
IX) Abführen des Reinigungswassers.

12. Verfahren nach Anspruch 11, **gekennzeichnet durch** folgende Schritte:
X) Spülen (142) des Präparationsbehälters (12) und der damit verbundenen Leitungen mit Frischwasser; und
XI) Abführen des Spülwassers.

13. Verfahren nach einem der Ansprüche 11 oder 12, **dadurch gekennzeichnet, dass** während des Schritts V) oder nach Schritt VI) ein Umpumpen (122) des Prozessabwassers vom Präparationsbehälter (12) in einen Konditionierungsbehälter (13) erfolgt und/oder dass beim Schritt IX) und/oder beim Schritt XI) das Abführen in den Konditionierungsbehälter (13) erfolgt und dass ein Neutralisierungsmittel, insbesondere eine Säure in den Konditionierungsbehälter zugeführt (121) wird, bis der pH-Wert des Abwassers einen Schwellenwert für den pH-Wert unterschritten hat und dann das Abwasser durch einen Abwasserabfluss (44) abgepumpt (145) wird.

## Claims

1. Apparatus for alkaline hydrolysis of corpses and/or cadavers, comprising:
- a processing tray (6) into which the corpse or the cadaver can be introduced;
- a hood (5) which encloses the processing tray (6);
- a preparation tank (12);
- at least one fresh water inlet (26, 27);
- at least one nozzle (16, 17), connectable to the preparation tank (12), in the hood (5) and/or in the processing tray (6);
- a wastewater outlet (44);
- at least one pump (15, 14); and
- a programmable logic controller (79) which controls the apparatus,
**characterised in that** a collecting tray (2) is arranged underneath the processing tray and **in that** the processing tray (6) is mounted so as to be able to move relative to the collecting tray (2) between a treatment position, in which the processing tray (6) is arranged above a treatment section (20) of the collecting tray (2) that is connected to the preparation tank (12), and a cleaning position, in which the processing tray (6) is arranged above a cleaning section (21) of the collecting tray (2) that is connected to the wastewater outlet (44).

2. Apparatus according to claim 1, **characterised by** a conditioning tank (13) which with the preparation tank (12) by at least one pipe through which a process wastewater is led to the conditioning tank (13), wherein the conditioning tank (13) has a device for neutralising the lye in the process wastewater.

3. Apparatus according to claim 2, **characterised in that** the device for neutralising the lye is a dosing device (42) for feeding liquid or solid acid into the conditioning tank (13).

4. Apparatus according to one of claims 2 or 3, **characterised in that** a first pump (14) is provided for conveying via the preparation tank (12) and/or a second pump (15) is provided for conveying via the conditioning tank (13).

5. Apparatus according to one of the preceding claims, **characterised in that** the hood (5) for the processing tray (6) is mounted so as to be able to move between an inserting position, in which the processing tray (6) is freely accessible from the outside, and a treatment position, in which the hood (5) encloses the processing tray (6).

6. Apparatus according to one of the preceding claims, **characterised in that** a multistage filter (24) having a replaceable filter unit is arranged in a drain for the process water.

7. Apparatus according to claim 6, **characterised in that** the replaceable filter unit is designed for single use and has an identifying mark to assign the corpse or cadaver.

8. Apparatus according to one of the preceding claims, **characterised by** at least one loading basket (7) which is configured to receive the corpse and/or cadaver and to be inserted into the processing tray (6).

9. Apparatus according to one of the preceding claims, **characterised by** a heating unit (66) which is connected to the preparation tank (12).

10. Apparatus according to one of the preceding claims, **characterised in that** at least one measuring device is provided for measuring one or more of the following parameters:
- flow rate of the process water;
- temperature of the process water;
- pH of the process water;
- amount of base or lye supplied;
- amount of water supplied;
- temperature of the process wastewater;
- pH of the process wastewater;
- conductivity of the process wastewater.

11. Method for alkaline hydrolysis of corpses and/or cadavers, consisting of the following steps:
I) supplying (102) a calculated amount of water through the at least one fresh water inlet (42) into a preparation tank (12);
II) introducing (105) lye and/or base into the preparation tank (12);
III) inserting (109) the corpse or cadaver in a processing tray (6);
IV) bringing (111) a hood (5) into a treatment position over the processing tray (6);
V) spraying (114) the corpse or cadaver through at least one nozzle (16, 17) in the hood and/or in the process tray (6) with process water from the preparation tank (12);
VI) draining off the process water;
VII) after the specified processing time, moving (130) the processing tray (6) into a cleaning position;
VIII) cleaning (134) the processing tray (6), the hood (5) and a collection tray (2);
IX) draining off the cleaning water.

12. Method according to claim 11, **characterised by** the following steps:
X) rinsing (142) the preparation tank (12) and the pipes connected thereto with fresh water; and
XI) draining of the rinsing water.

13. Method according to one of claims 11 or 12, **characterised in that** during step V) or after step VI), the process wastewater is pumped (122) from the preparation tank (12) into a conditioning tank (13), and/or **in that** in step IX) and/or in step XI), draining into the conditioning tank (13) takes place and **in that** a neutralising agent, in particular an acid, is supplied (121) into the conditioning tank until the pH of the wastewater has dropped below a pH threshold value, and then the wastewater is pumped out (145) through a wastewater outlet (44).

## Revendications

1. Dispositif pour l'hydrolyse alcaline de cadavres et/ou de carcasses, comprenant :
- une cuve de traitement (6) dans laquelle le cadavre ou la carcasse peuvent être placés,
- un capot (5) qui entoure la cuve de traitement (6),
- un bac de préparation (12),
- au moins une arrivée d'eau fraîche (26, 27),
- au moins une buse (16, 17) dans le capot (5) et/ou dans la cuve de traitement (6), qui peut être reliée au bac de préparation (12),
- une sortie d'eaux usées (44),
- au moins une pompe (15, 14), et
- un organe de commande programmable (79) qui commande le dispositif,
**caractérisé en ce qu'**une cuve collectrice (2) est agencée en dessous de la cuve de traitement (6) et **en ce que** la cuve de traitement(6) est montée mobile par rapport à la cuve collectrice (2) entre une position de traitement dans laquelle la cuve de traitement (6) est agencée au-dessus d'une section de traitement (20) de la cuve collectrice (2) qui est reliée au bac de préparation (12), et une position de nettoyage dans laquelle la cuve de traitement (6) est agencée au-dessus d'une section de nettoyage (21) de la cuve collectrice (2) qui est raccordée à la sortie d'eaux usées (44).

2. Dispositif selon la revendication 1, **caractérisé par** un bac de conditionnement (13) qui est relié au bac de préparation (12) par au moins une conduite permettant d'apporter au bac de conditionnement (13) des eaux usées de traitement, le bac de conditionnement (13) présentant un dispositif de neutralisation de la solution alcaline contenue dans les eaux usées de traitement.

3. Dispositif selon la revendication 2, **caractérisé en ce que** le dispositif de neutralisation de la solution alcaline est un dispositif de dosage (42) permettant d'introduire de l'acide liquide ou solide dans le bac de conditionnement (13).

4. Dispositif selon l'une des revendications 2 et 3, **caractérisé en ce qu'**il est prévu une première pompe (14) de circulation concernant le bac de préparation (12) et/ou une deuxième pompe (15) de circulation concernant le bac de conditionnement (13).

5. Dispositif selon l'une des revendications précédentes, **caractérisé en ce que** le capot (5) est monté mobile par rapport à la cuve de traitement (6) entre une position de mise en place dans laquelle la cuve de traitement (6) est librement accessible depuis l'extérieur et une position de traitement dans laquelle le capot (5) entoure la cuve de traitement (6).

6. Dispositif selon l'une des revendications précédentes, **caractérisé en ce qu'**un filtre à plusieurs étages (24) est agencé dans une voie d'évacuation d'eau de traitement et comporte une unité de filtrage remplaçable.

7. Dispositif selon la revendication 6, **caractérisé en ce que** l'unité de filtrage remplaçable est conçue pour un usage unique et comporte une marque d'identification permettant de l'associer au cadavre ou à la carcasse.

8. Dispositif selon l'une des revendications précédentes, **caractérisé par** au moins une nacelle de chargement (7) conçue pour recevoir le cadavre et/ou la carcasse et pour être mise en place dans la cuve de traitement (6).

9. Dispositif selon l'une des revendications précédentes, **caractérisé par** un élément chauffant (66) relié au bac de préparation (12).

10. Dispositif selon l'une des revendications précédentes, **caractérisé en ce qu'**il est prévu au moins un dispositif de mesure propre à un ou plusieurs des paramètres suivants :
- débit de l'eau de traitement,
- température de l'eau de traitement,
- valeur du pH de l'eau de traitement,
- quantité de la base ou de la solution alcaline apportée,
- quantité d'eau apportée,
- température des eaux usées de traitement,
- valeur du pH des eaux usées de traitement,
- conductivité des eaux usées de traitement.

11. Procédé d'hydrolyse alcaline de cadavres et/ou de carcasses, comprenant les étapes suivantes consistant à :
I) apporter (102) une quantité mesurée d'eau à un bac de préparation (12) par l'au moins une entrée d'eau fraîche (42),
II) introduire (105) une solution alcaline et/ou une base dans le bac de préparation (12),
III) mettre en place (109) le cadavre ou la carcasse dans une cuve de traitement (6),
IV) mettre (111) un capot (5) en position de traitement au-dessus de la cuve de traitement (6),
V) pulvériser (114) de l'eau de traitement provenant du bac de préparation (12) sur le cadavre ou la carcasse par le biais d'au moins une buse (16, 17) présente dans le capot et/ou dans la cuve de traitement (6),
VI) évacuer les eaux de traitement,
VII) après le temps de traitement prescrit, faire passer (130) la cuve de traitement (6) en position de nettoyage,
VIII) nettoyer (134) la cuve de traitement (6), le capot (5) et une cuve collectrice (2),
IX) évacuer les eaux de nettoyage.

12. Procédé selon la revendication 11, **caractérisé par** les étapes suivantes consistant à :
X) rincer (142) le bac de préparation (12) et les conduites qui y sont raccordées avec de l'eau fraîche, et
XI) évacuer l'eau de rinçage.

13. Procédé selon l'une des revendications 11 et 12, **caractérisé en ce que**, pendant l'étape V) ou après l'étape VI), il s'effectue un pompage (122) des eaux usées de traitement depuis le bac de préparation (12) en direction d'un bac de conditionnement (13) et/ou **en ce que**, pendant l'étape IX) et/ou l'étape XI), l'évacuation s'effectue en direction du bac de conditionnement (13) et **en ce qu'**un agent neutralisant, notamment un acide, est apporté (121) au bac de conditionnement jusqu'à ce que la valeur du pH des eaux usées tombe en dessous d'une valeur seuil concernant ladite valeur du pH puis les eaux usées sont évacuées par pompage (145) par une sortie d'eaux usées (44).
